(19) **Europäisches Patentamt European Patent Office Office européen des brevets**

(11) **EP 4 560 295 A1**

(12) **EUROPEAN PATENT APPLICATION**
published in accordance with Art. 153(4) EPC

(43) Date of publication:
28.05.2025 Bulletin 2025/22

(21) Application number: 24845175.9

(22) Date of filing: 24.05.2024

(51) International Patent Classification (IPC):
*G01N 21/3563* (2014.01)     *G01N 21/27* (2006.01)
*G01N 21/359* (2014.01)

(52) Cooperative Patent Classification (CPC):
G01N 21/01; G01N 21/27; G01N 21/31;
G01N 21/3563; G01N 21/359

(86) International application number:
PCT/JP2024/019174

(87) International publication number:
WO 2025/022793 (30.01.2025 Gazette 2025/05)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 24.07.2023 JP 2023120079

(71) Applicant: Atago Co., Ltd.
Minato-ku, Tokyo 105-0011 (JP)

(72) Inventors:
• AMAMIYA Hideyuki
  Tokyo 105-0011 (JP)
• TANAKA Masanosuke
  Tokyo 105-0011 (JP)
• TERASAWA Yuichi
  Tokyo 105-0011 (JP)

(74) Representative: Kuhnen & Wacker
Patent- und Rechtsanwaltsbüro PartG mbB
Prinz-Ludwig-Straße 40A
85354 Freising (DE)

(54) **NON-DESTRUCTIVE MEASUREMENT DEVICE AND NON-DESTRUCTIVE MEASUREMENT METHOD FOR FRUITS AND VEGETABLES**

(57) Disclosed is a nondestructive measurement apparatus and method for fruit or vegetable by which a plurality of internal information therein even if the skin is thick is obtained. A nondestructive measurement apparatus includes a casing including a grip portion capable of being held in one hand and a measurement portion having a ring shaped abutting portion to be abutted to a measurement target such as fruit or vegetable; a light source group including a plurality of light sources arranged separately in a circumferential direction in an interior thereof; a light intensity controller for controlling the amount of light emitted from the light source group, the light intensity controller controlling the light source group to emit at least two different light amount; a ring lens arranged in a ring shape smaller than the abutting portion at an inner portion of the abutting portion, for emitting lights from the light source group to an external of the casing in a ring shape; and a light guide member having one end surface exposed to an inner side of the ring lens and another end surface positioned in the interior of the casing, for emitting lights incident from the one end surface to the external from the another end surface; a photo sensor arranged in the casing, for receiving light emitted from the another end surface of the light guide member; and a light intensity processor for obtaining absorbance based on the light intensity from the photo sensor.

**Fig. 4**

**Description**

TECHNICAL FIELD

The present invention relates to a nondestructive measurement apparatus and a method for nondestructively measuring absorbance of a measurement target such as fruit or vegetable.

BACKGROUND TECHNIQUE

A nondestructive measurement apparatus for fruit or vegetable, for measuring absorbance of fruit or vegetable nondestructively has been known. As an example, there is a nondestructive measurement apparatus which measures absorbance by utilizing a transmission light of a near infrared light irradiated and injected into the fruit or vegetable, and obtains a sugar content of the fruit or vegetable as a Brix value, according to the measured absorbance. This nondestructive measurement apparatus is disclosed as the nondestructive sugar content measurement apparatus in Patent Document 1.

The nondestructive measurement apparatus disclosed in Patent Document 1 is one that measures the fruit or vegetable after the harvest as a target. Also, the apparatus is of large size, as the absorbance is measured by mounting the harvested fruit or vegetable on a conveyor.

From the viewpoint of producers of the fruit or vegetable, there are demands for an apparatus that can take not only the harvested fruit or vegetable but also the fruit or vegetable in a state of being born on a tree or the like (growing) before the harvest as a target, and that can comprehend the sugar content by measuring absorbance nondestructively, for the purpose of ascertaining the harvest period and the like. More specifically, there are demands for a nondestructive measurement apparatus that is compact to a level capable of being held in one hand, so that it can be easily used even for the growing fruit or vegetable.

Also, in the case of making the nondestructive measurement apparatus compact to a level capable of being held in one hand, the adoption of LEDs (Light Emitting Diodes) will be considered, from viewpoints of saving space and saving electricity consumption.

In Patent Document 2, there is disclosed a nondestructive measurement apparatus for measuring nondestructively absorbance of such target as fruit, wherein LEDs are provided therein as the light source.

In nondestructive measurement of fruit, precise measurement of absorbance information of light passing inside the fruit by irradiating near infrared light thereon is necessary. However, if the target is big object such as melon or citrus fruits, as skin is thick, light does not enter sufficiently inside the object. As a result, it is difficult to obtain absorbance information relating to the sugar content in the object. In order to let light effectively inside the object, it is necessary to use a light source which has a high intensity of light. However, with conventional method of driving LEDs, available light intensity is too low, so there is a problem of insufficient light entering inside the fruit or vegetables.

In order to obtain a calibration curve of sugar content of fruit or vegetable, it is necessary to obtain a certain range of sugar content information from the fruit or vegetable to be measured. However, as to watermelon, for example, because the sugar content at the center portion thereof is substantially constant and further an individual difference is small or little, even if many samples are prepared and are subjected to measurement operation, the measured values are concentrated in one point, whereby it is difficult to obtain an effective calibration curve.

PRIOR ART DOCUMENT

Patent Document

    Patent Document 1: Japanese Patent Application Publication No. 2002-014042
    Patent Document 2: WO2018-047366

SUMMARY OF THE INVENTION

Therefore, the object of the present invention is to provide a nondestructive measurement apparatus and method, with which a plurality of information in the direction of depth of the targeted fruit or vegetable, and inside information of thick skin fruit or vegetable.

According to one aspect of the invention, there is provided a nondestructive measurement apparatus for fruit or vegetable, comprising:

a casing including a grip portion which is held by a hand of an operator, and a measurement portion having a ring shaped abutting portion which is configured to be abutted to a fruit or vegetable;

a light source group including a plurality of light sources which are arranged separately in a circumferential direction in an interior of the casing;

a light amount control portion for controlling the light amount emitted from the light source group;

a ring lens arranged in a ring shape smaller than an inner portion surrounded by said abutting portion, for emitting light coming from the light source group toward an outside of the casing as ring shape light as a ring shaped beam;

a light guide member having one end surface exposed to an inner side of the ring lens and the other end surface positioned in the interior of the casing, the light guide member being configured to emit light, for emitting the light entered from the one end surface to the outside from the other end surface;

photo sensors arranged inside the casing, for detecting light emitted from the other end surface of the light guide member; and

a light intensity processor for obtaining an absorbance according to detected intensities of the photo sensors, wherein the light source group is controlled by the light amount control portion to emit light of at least two different light amount, and

wherein the light intensity processor obtains a calibration curve of sugar content of the fruit or vegetable based on the absorbance of the at least two different light amount and Brix value of the fruit or vegetable in a depth direction corresponding to the light amount.

In the nondestructive measurement apparatus, the light intensity processor may obtain a Brix value distribution in the depth direction of the fruit or vegetable based on absorbance of the at least two light amount of Brix value.

In the nondestructive measurement apparatus, the light intensity processor may obtain a calibration curve of sugar content of the fruit or vegetable, based on absorbance of the at least two different light amount and the Brix value of the corresponding to the two light amount of the fruit or vegetable.

The nondestructive measurement apparatus may further comprise an electric circuit having an electrolytic capacitor which is charged from a power source while the light source group does not emit light and, when the light source group emits light, both the electrolytic capacitor and the power source supply currents to the light source group

The nondestructive measurement apparatus may comprise a ring shaped relay lens between the light source group and the ring lens, for guiding the light from the light source group to the ring lens.

In the nondestructive measurement apparatus, the main optical axis of the ring shaped light emitted from the ring lens may incline in the direction of reducing diameter after the light is emitted from the ring lens.

In the nondestructive measurement apparatus, the photo sensors may be formed by at least m sets of photo sensors (m being an integer greater than or equal to 2), and the apparatus may further comprise band-pass filters, each having a respective one of m types of different wavelengths $\lambda 1$-$\lambda m$ as a central wavelength, between a respective one of the m sets of the photo sensors and the other end surface of the light guide member.

In the nondestructive measurement apparatus, the light intensity processor may obtain absorbance according to the detected light intensities corresponding to the wavelengths $\lambda 1$-$\lambda m$ obtained by the m sets of photo sensors, and it may calculate Brix value from the obtained absorbance.

In the nondestructive measurement apparatus, the grip portion has a longitudinal handle which is holdable by a hand of an operator, and the measurement portion may be formed such that an extending direction of the outer abutting portion is a direction along the handle, at one end portion of the handle in the grip portion, and a tip end portion of the outer abutting portion is positioned to protrude from a surface of the grip portion.

The nondestructive measurement apparatus may further comprise a light diffusion member between the other surface of said light guide member and a surface of the fruit or vegetable surrounded by the abutting portion while the abutting portion and the fruit or vegetable are kept in contact under measurement, for diffusing the light passing therethrough.

According to another aspect of the invention, there is provided a nondestructive measurement method for fruit or vegetable, comprising the steps of: contacting fruit or vegetable to be measured to a measurement portion; irradiating multiple light of at least two light amount onto said fruit or vegetable so that light emitted from a light source group formed by a plurality of light sources enters inside the fruit or vegetable; receiving, by a photo sensor, light outputted from the fruit or vegetable after subjected to diffused reflection and partial absorption in the fruit or vegetable, due to the internal condition of the fruit or vegetable; and processing light intensity to obtain absorbance based on light intensity detected by said photo sensor.

In the nondestructive measurement method, the step of processing light intensity may include a step of obtaining a distribution of Brix values in the direction of depth of the fruit or vegetable, based on the at least two different light amount.

In the nondestructive measurement method, the step of processing light intensity may include a step of obtaining a calibration curve of sugar content of the fruit or vegetable, based on the absorbance of the at least two different light amount and the Brix value in the direction of the fruit or vegetable corresponding to the light amount.

In the nondestructive measurement method, the step of multiple irradiating light may further comprise a step of charging an electrolytic capacitor at non-irradiating time and supplying currents to a light source group from both the power source and the electrolytic capacitor while the light source group emits light.

The nondestructive measurement apparatus and method of fruit or vegetable according to the present invention make it possible to obtain a plurality of information in its depth direction and to obtain the necessary internal information for even thick skin fruit or vegetable.

BRIEF DESCRIPTION OF THE DRAWINGS

FIG. 1 is a perspective view of an outward appearance of a handy nondestructive saccharimeter 51 (saccharinity meter 51) which is one embodiment of the nondestructive measurement apparatus according to the present invention.

FIG. 2 is a front view of the saccharinity meter 51 of FIG. 1.

FIG. 3 is a cross sectional view at S3-S3 position in FIG. 2.

FIG. 4 is an assembly diagram of the saccharinity meter 51 of FIG. 1.

FIG. 5 is a front view of a sensor substrate 13 equipped by the saccharinity meter 51 of FIG. 1.

FIG. 6 is a front view of a base substrate 14 equipped by the saccharinity meter 51 of FIG. 1.

FIG. 7 is a partial cross sectional view for explaining an attaching state of a relay lens 18 equipped by the saccharinity meter 51 of FIG. 1.

FIG. 8 is a half cross sectional view of a ring lens 8 equipped by the saccharinity meter 51 of FIG. 1.

FIG. 9 is an assembly diagram for explaining a filter unit F equipped by the saccharinity meter 51 of FIG. 1.

FIG. 10 is a rear view of the filter unit F of FIG. 9.

FIG. 11 is a perspective view of a light guide member 11 equipped by the saccharinity meter 51 of FIG. 1.

FIG. 12 shows two views of the light guide member 11 of FIG. 11, where (a) is a half cross sectional side view and (b) is a rear view.

FIG. 13 is an enlarged cross sectional view of a SB section in FIG. 3.

FIG. 14 is a diagram for explaining a control system of the saccharinity meter 51 of FIG. 1.

FIG. 15 is a diagram for explaining a manner of measurement by the saccharinity meter 51 of FIG. 1 by mounting on top of table.

FIG. 16 is a diagram for explaining a manner of measurement by the saccharinity meter 51 of FIG. 1 by being held in hand.

FIG. 17 is a partial cross sectional view for explaining light passages in the saccharinity meter 51 of FIG. 1.

FIG. 18 is a half cross sectional view for explaining a standard lid body 52 to be used for a calibration of the saccharinity meter 51 of FIG. 1 and its state of use.

FIG. 19 is a partial cross sectional view for explaining a diffusion plate 41 as a modified example 1 of the saccharinity meter 51 of FIG. 1 and its attaching manner.

FIG. 20 is a half cross sectional view for explaining a light guide member 11W as a modified example 2 of the saccharinity meter 51 of FIG. 1.

FIG. 21 is a view showing a cut watermelon with four portions indicated by A to D (from a center to a skin portion of the watermelon).

FIG. 22 is a graph showing the results of multiple regression analysis with measured Brix values.

FIG. 23 is a graph showing the results of multiple regression analysis with measured Brix values.

FIG. 24 is a diagram showing an electric circuit for supplying power to light sources from power source and electrolytic capacitor.

EMBODIMENTS FOR IMPLEMENTING THE INVENTION

The nondestructive measurement apparatus according to the present invention will now be explained by taking an example of a handy-type nondestructive saccharimeter 51 (also referred to simply as saccharinity meter 51 in the following) which is its one embodiment.

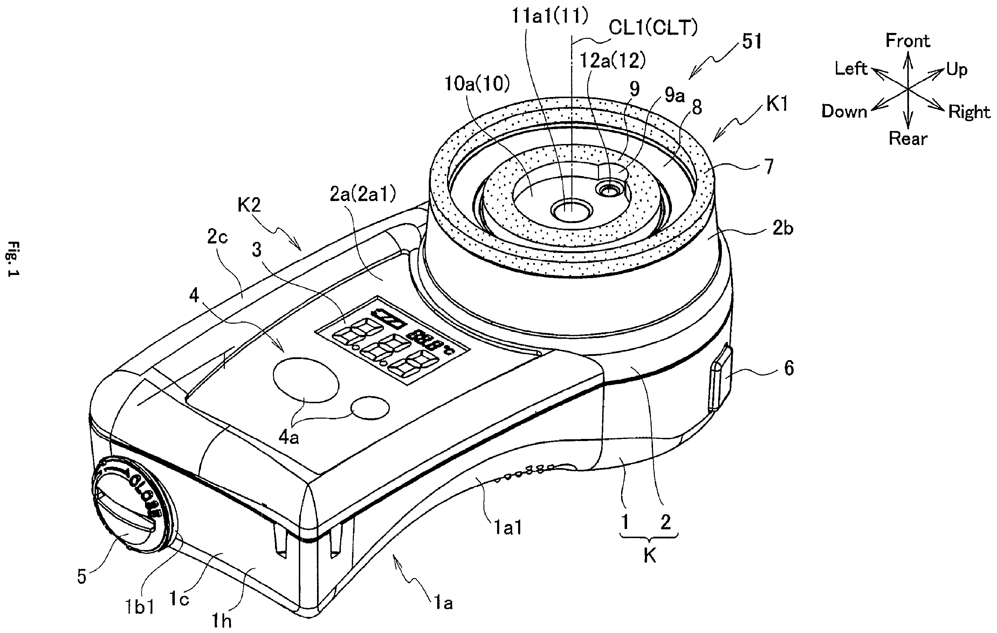

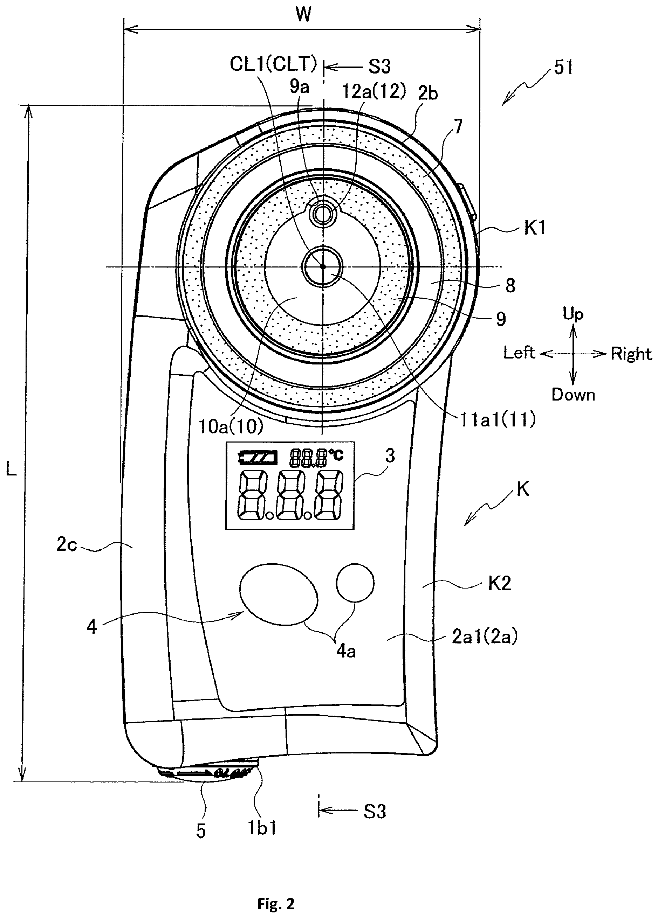

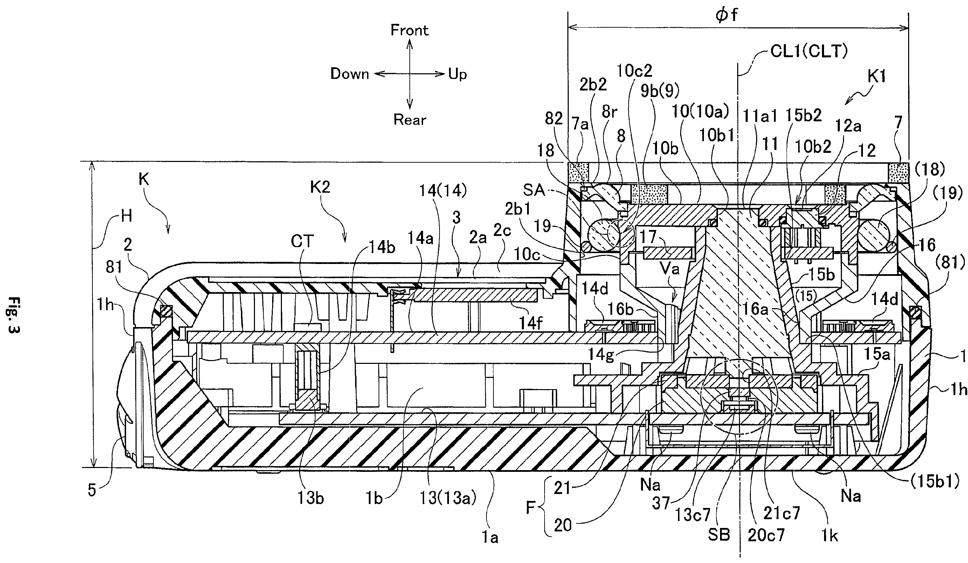

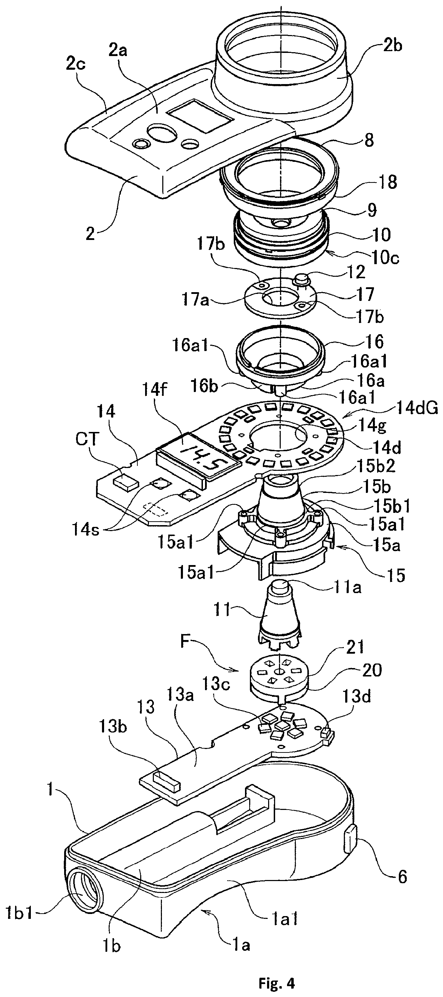

First, the configuration of the saccharinity meter 51 will be described with references to FIG. 1 to FIG. 4. FIG. 1 is a perspective view of an outward appearance of the saccharinity meter 51. FIG. 2 is a front view of the saccharinity meter 51. FIG. 3 is a cross sectional view at S3-S3 position in FIG. 2. FIG. 4 is an assembly diagram of the saccharinity meter 51.

In the following explanation, respective directions of up, down, left, right, front and rear are defined to be directions shown in FIG. 1. The saccharinity meter 51 is of the so called handy type, which is capable of being held in one hand of an operator. Consequently, up, down, left, right, front and rear directions shown in FIG. 1 are defined for the convenience of the explanation, and not limiting a posture and the like of the saccharinity meter 51 at a time of use.

First, an outward appearance configuration of the saccharinity meter 51 will be described. The saccharinity meter 51 has an approximately box shaped box body 1 with its front side open, and an approximately lid shaped lid body 2 to be attached to the box body 1 so as to close the front side of the box body 1. As shown in FIG. 3, the box body 1 has a base portion 1k formed to be roughly flat plate shape, and a side wall portion 1h that is erecting in front from a surrounding of the base portion 1k, and is made to be a box shape.

The box body 1 and the lid body 2 constitute a casing K that is integrated with an O-ring 81 (see FIG. 3) intervened therebetween, by tapping screws (not shown in the figure). The saccharinity meter 51 has the water-proof and dust-proof function of IP65 or above, which is the protection characteristic in the IEC (International Electrotechnical Commission) standard, by the mediation of the O-ring 81 and the other sealing structures not shown in the figure and the like in the casing K.

The casing K has a measurement portion K1 of an approximately cylindrical shape centered around an axial line CL1 extending in the front and rear direction, which is formed on an upper portion, and a grip portion K2 in an approximately flat and rectangular parallelepiped shape extending downward from a rear portion side of the measurement portion K1.

The lid body 2 has a front surface portion 2a having a roughly flat front surface 2a1 in the grip portion K2, and a stage portion 2b protruding forward in a cylindrical shape with respect to a front and rear direction position of the front surface portion 2a in the measurement portion K1. The lid body 2 has a bank portion 2c protruding forward at a left edge portion of the front surface portion 2a. The bank portion 2c is a portion to be a finger hook in a hand held state to be described below.

The axial line CL1 of the measurement portion K1 is coinciding with a light receiving axial line CLT to be described below. The light receiving axial line CLT is virtually set up as an optical axis of light to be received by photo sensors 13c to be described below.

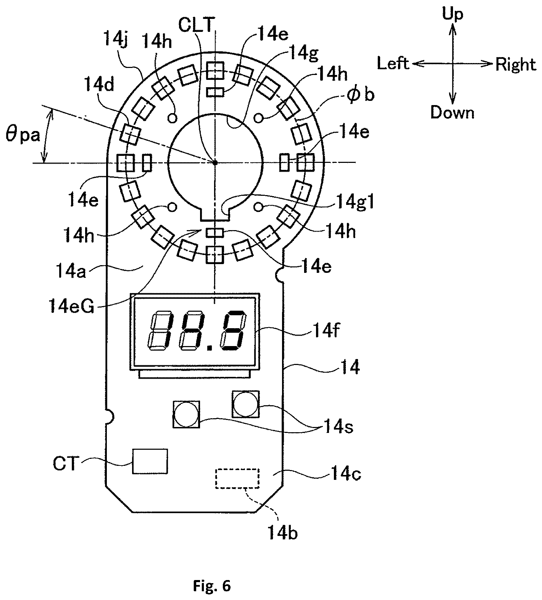

In the grip portion K2, on the front surface 2a1 of the lid body 2, there are provided a display unit 3 for displaying numerals, letters and signs to be visually recognizable by a display element 14f (see FIG. 3 and FIG. 6), and a switch unit 4 containing a plurality of switch pressing portions 4a for selecting an operation mode and zero resetting by pressing with a finger.

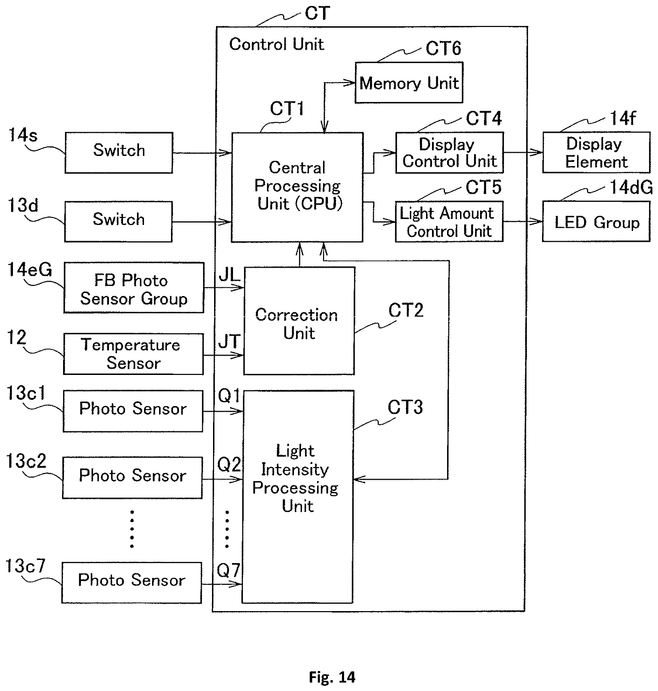

The display unit 3 displays an operation state of the saccharinity meter 51, a battery remaining amount, and a sugar content (Brix value) obtained by the measurement, for example, under the control of a control unit CT (see FIG. 3 and FIG. 14).

On a left rear portion in the interior of the box body 1, a battery box 1b (see FIG. 3) having an outlet/inlet 1b1 on a lower surface 1c, for accommodating a battery is formed. To the outlet/inlet 1b1, a battery lid 5 is attached to be freely detachable. In the battery box 1b, a size AAA battery, for example, is accommodated to be capable of being inserted and removed from the outlet/inlet 1b1 by a user.

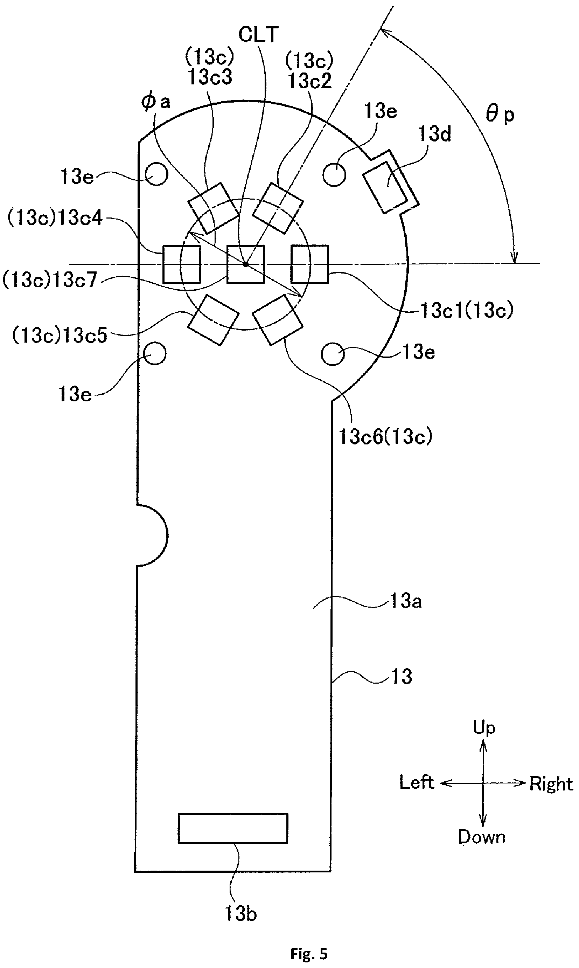

On an upper right portion of the box body 1, a press button 6 is provided. Upon pressing the press button 6 by the user, a switch 13d (see FIG. 4 and FIG. 5) provided in the interior is activated and operations of start and stop of the measurement are executed alternately.

The grip portion K2 is formed in a size that can be held by one hand of operator. At a time of holding the grip portion K2 with a right hand, for example, in order to use this saccharinity meter 51, when a palm is touched to a rear surface 1a of the box body 1, four fingers from an index finger to a little finger will naturally be hooked by the bank portion 2c, so that the operator can hold the saccharinity meter 51 well. In this holding state, the press button 6 is provided at a position that is easy to press by a thumb. Also, on a right portion of the rear surface 1a of the box body 1, a padding portion 1a1 (see FIG. 1) of a concave curving surface in accordance with a thenar is formed so that a thenar portion of the hand will touch with a good feel.

The box body 1 and the lid body 2 are formed by resin. The resin is a near infrared absorption grade of a black polycarbonate resin, for example.

Next, an outward appearance configuration of the measurement portion K1 will be described in detail.

To a tip end of the stage portion 2b in the lid body 2, a ring shaped outer abutting portion 7 is attached. The outer abutting portion 7 is formed by a material having elasticity with respect to at least a compression to rearward. An example of the material is a sponge.

In the front side view shown in FIG. 2, a ring lens 8 that is a lens member, an inner abutting portion 9, and a part of a front surface 10a of a stage base 10 are visible in respective ring shapes, sequentially from an outer side, in an inner side portion surrounded by the outer abutting portion 7. In a central portion containing the axial line CL1, a front end surface 11a1 of a light guide member 11 is visible.

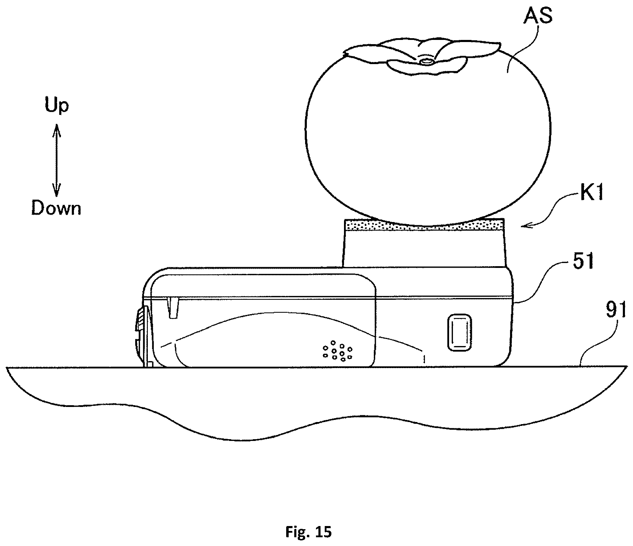

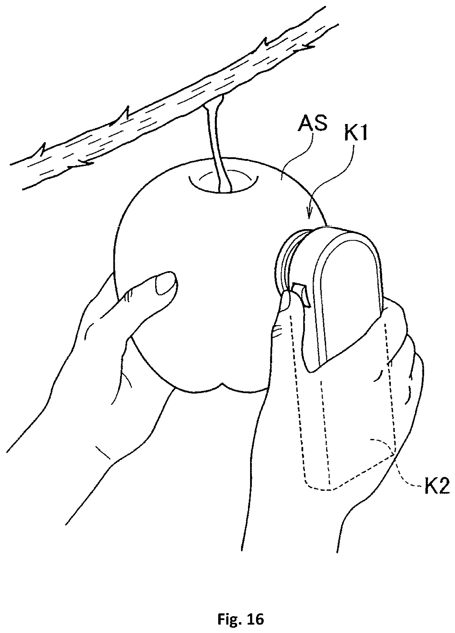

The inner abutting portion 9 is formed in a ring shape, and attached to the front surface 10a. The inner abutting portion 9 is formed by a material having elasticity with respect to at least a compression to rearward. An example of the material is a sponge. The inner abutting portion 9 has a cut out portion 9a curved in an arc shape to outward in a radial direction, at a part of an inner edge. To the stage base 10, a temperature sensitive surface 12a of a temperature sensor 12 is attached to be exposed to a front side. The temperature sensor 12 is arranged such that a part of the temperature sensitive surface 12a is entering within the cut out portion 9a, when viewed from a front. The temperature sensor 12 is the so called thermopile, which measures a temperature T2 in a noncontact manner of a surface of the fruit or vegetable AS (see FIG. 15 and FIG. 16) that is a measurement target put in contact with the measurement portion K1, and measures a temperature T1 corresponding to the casing K in the surrounding as well. The temperature sensor 12 outputs the measured temperature T1 and temperature T2 toward the control unit CT as temperature information JT (see FIG. 14). The temperature T1 and the temperature T2 may be reversed. Namely, the temperature of the surface of the fruit or vegetable AS may be set as the temperature T1. In the casing K, the measurement portion K1 is integrally formed on one end side (an upper side) of the grip portion K2, in such a posture that an extended surface in a ring shape of the outer abutting portion 7 and the inner abutting portion 9 becomes a surface along the up and down direction that is a length direction of the grip portion K2 (to be roughly parallel, for example).

As shown in FIG. 3, a position in the front and rear direction of each portion in the measurement portion K1 is such that a front end surface 9b of the inner abutting portion 9 is on a rear side, with respect to a front end surface 7a of the outer abutting portion 7. The ring lens 8 is arranged in the roughly same front and rear position as the inner abutting portion 9. A front end ridgeline portion 8r positioned at a front-most side in the ring lens 8 is in the roughly same front and rear position as the front end surface 9b of the inner abutting portion 9. The front surface 10a of the stage base 10 is on a rear side, with respect to the front end surface 9b of the inner abutting portion 9. The front end surface 11a1 of the light guide member 11 is positioned on the same position in the front and rear direction or on a slightly rear side, with respect to the front surface 10a of the stage base 10.

Next, parts and the like arranged in the interior of the casing K will be described with reference to FIG. 3 to FIG. 12. As shown in FIG. 3 and FIG. 4, in the interior of the casing K, two large substrates are accommodated in parallel, to be facing against each other in the front and rear direction. More specifically, they are a sensor substrate 13 and a base substrate 14 from the base portion 1k side of the box body 1.

FIG. 5 is a front view for explaining the sensor substrate 13. The sensor substrate 13 has a receptacle 13b, a plurality of photo sensors 13c, and a switch 13d implemented on a front surface 13a to be a front side in a state of being accommodated within the casing K.

The receptacle 13b is installed on a plug 14b of the base substrate 14 arranged in front of the sensor substrate 13, such that the sensor substrate 13 and the base substrate 14 are electrically connected (see FIG. 3).

The plurality of photo sensors 13c are provided to be seven in this example. Six of the photo sensors 13c among seven of them are implemented such that they are equally distanced by an angle pitch $\theta p$ of 60° around the light receiving axial line CLT, such that a central position of each photo sensor 13c is on a line of a diameter $\phi a$. The remaining one photo sensor is implemented at a central position of the diameter $\phi a$. These seven photo sensors 13c will be distinguished as photo sensors 13c1 to 13c7, as shown in FIG. 5, in the case where a distinction becomes necessary in the later explanation.

The switch 13d alternately repeats an ON operation and an OFF operation whenever the press button 6 is pressed as already described, in the assembled saccharinity meter 51.

The sensor substrate 13 has four piercing holes 13e at approximately 90° interval, at positions on outer side in the diameter direction than the photo sensors 13c.

FIG. 6 is a front view for explaining the base substrate 14. The base substrate 14 has a circular hole 14g centered around the light receiving axial line CLT on an upper portion, a cut out portion 14g1 curved in a rectangular shape toward downward, and holes 14h at an outer side of the hole 14g in oblique 45° directions with respect to up, down, left and right, respectively. The base substrate 14 has an arc portion 14j to be an arc shaped outer shape centered around the light receiving axial line CLT.

The base substrate 14 has the display element 14f, a switch 14s, a plurality (n sets; where n is an integer greater than or equal to 2) of LEDs 14d as the light emitting elements to be the light source, a plurality of FB (feedback) photo sensors 14e, and the control unit CT for controlling operations of the saccharinity meter 51, implemented on a front surface 14a to be a front side in a state of being accommodated within the casing K. On the other hand, on a rear surface 14c, the plug 14b to be connected with the receptacle 13b of the sensor substrate 13 is implemented.

The display element 14f is a display device such as a liquid crystal device, an organic EL (organic Electro-Luminescence) device and the like, for example.

The plurality of LEDs 14d are provided to be twenty (n=20) in this example. These 20 LEDs 14d are implemented in a posture to be radial, such that they are equally distanced by an angle pitch $\theta pa$ of 18° on a line of a diameter $\theta b$ around the hole 14g centered around the light receiving axial line CLT. In detail, the LEDs 14d are arranged to be separated in a circumferential direction at positions close to the arc portion 14j. In the following, they are also referred to as a LED group 14dG, as a light source group collecting the plurality of LEDs 14d together.

The LEDs 14d are arranged sequentially in the circumferential direction, using 6 types having the following wavelengths as respective central wavelengths, for example. Namely, these wavelengths are 880 nm, 900 nm, 950 nm, 980 nm, 1020 nm, and 1064 nm. In this case, at least three sets of those LEDs 14d that have each wavelength as the central wavelength will be arranged in the entire 20 sets. Among them, respectively four sets of those LEDs 14d that have 880 nm and 900 nm as respective central wavelengths will be arranged. The selection of the LEDs 14d with respect to 6 types of wavelengths is not limited to this. As another example, it may use the so called 3-wavelength compound LEDs, in which 3 types of LEDs having 3 types of wavelengths as respective central wavelengths are packaged into one, for 3 types of short wavelengths and 3 types of long wavelengths among 6 types of wavelengths. For example, using the 3-wavelength compound LEDs having 3 types of wavelengths 880, 900 and 950 nm as the central wavelengths, and the 3-wavelength compound LEDs having 3 types of wavelengths 980, 1020 and 1064 nm as the central wavelengths, a total 20 sets including 8 sets of the former and the 12 sets of the latter, for example, may be arranged appropriately.

Between an edge portion of the hole 14g and the LEDs 14d, the FB (feedback) photo sensors 14e (hereafter referred to as the FB photo sensors 14e) are implemented in up, down, left and right directions, respectively. In the following, these four FB photo sensors 14e will also be referred to collectively as a FB photo sensor group 14eG.

As shown in FIG. 3 and FIG. 4, with respect to the sensor substrate 13, a flat cylindrical shaped filter unit F is arranged to cover seven sets of the photo sensors 13c. On a front side of the filter unit F, the light guide member 11 formed to be solid and roughly truncated cone shape is arranged.

The filter unit F and the light guide member 11 are provided on the sensor substrate 13 from a front side, and along with a unit holder 15, sandwiched between the sensor substrate 13 and a stage base mount 16 that is fastened and fixed by tapping screws Na from a rear side. A specific configuration of the light guide member 11, the stage base mount 16, and the filter unit F will be described below.

As shown in FIG. 3 and FIG. 4, the unit holder 15 has a base portion 15a in a round pot shape with a rear side open and a front side becoming a bottom, and a protruding portion 15b protruding to forward from the base portion 15a. On the base portion 15a, four piercing holes 15a1 (see FIG. 4) extending in the front and rear direction in order to insert the tapping screws Na are formed, in oblique 45° directions with respect to up, down, left and right. The protruding portion 15b is formed in a hollow cone shape, such that the light guide member 11 can be fit inside almost without any gap, and protruding to forward through the hole 14g of the base substrate 14.

On an outer circumferential surface of the protruding portion 15b, a step portion 15b1 and a step portion 15b2 whose diameters changes suddenly at two locations on a rear side and a tip end side are formed.

With respect to an inner diameter of the hole 14g of the base substrate 14, an outer diameter of the protruding portion 15b is set smaller, and a rear end portion 16a of the stage base mount 16 is engaged between the hole 14g and the protruding portion 15b. The rear end portion 16a has a front and rear direction position determined by abutting to the step portion 15b1 of the protruding portion 15b.

The stage base mount 16 is formed in an approximately funnel shape with a tapering rear end portion side. On the rear end portion 16a, a passage portion 16b protruding to a lower side is formed. An outer shape of the passage portion 16b is made to be engaged with the cut out portion 14g1 in the hole 14g of the base substrate 14. Namely, with respect to the base substrate 14, a position is determined around the light receiving axial line CLT of the stage base mount 16.

An inner circumferential surface of the rear end portion 16a is abutted to an outer surface of the protruding portion 15b of the unit holder 15, except for a portion of the passage portion 16b. Namely, a gap Va (see FIG. 3) is formed between the passage portion 16b and the protruding portion 15b. This gap Va will become a passage for passing a lead wire from a temperature sensor substrate 17 to be described below, in the front and rear direction.

On the rear end portion 16a, four bosses 16a1 extending in front and rear are formed in oblique 45° directions with respect to up, down, left and right in the front view. In each boss 16a1, a bottomed hole with a front side as a bottom is formed.

By inserting the tapping screws Na shown in FIG. 3 into the piercing holes 13e of the sensor substrate 13 and the piercing holes 15a1 of the unit holder 15, and screwing them into the bottomed holes formed in the bosses 16a1 of the stage base mount 16, the unit holder 15 and the stage base mount 16 are fixed with respect to the sensor substrate 13. At that point, the filter unit F and the light guide member 11 are held by being sandwiched between the sensor substrate 13 and the unit holder 15.

In front of the stage base mount 16, the stage base 19 is arranged. The stage base 10 has a disk shaped stage bottom portion 10b (see FIG. 3), and a ring shaped circumferential wall portion 10c erecting rearward from a periphery of the stage bottom portion 10b. The front surface 10a already described is a front surface of the stage bottom portion 10b.

As shown in FIG. 3, at a center (a position of the light receiving axial line CLT) of the stage bottom portion 10b, a piercing hole 10b1 is formed. Also, on an upper side with respect to the piercing hole 10b1, a piercing hole 10b2 is formed.

Into the piercing hole 10b1, a tip end portion of the light guide member 11 is entered from a rear side, and the front end surface 11a1 of the light guide member 11 is exposed forward. Into the piercing hole 10b2, the temperature sensor 12 is entered from a rear side, and the temperature sensitive surface 12a is exposed forward.

In the interior surrounded by the circumferential wall portion 10c of the stage base 10, the temperature sensor substrate 17 is arranged. As shown in FIG. 4, the temperature sensor substrate 17 is in a disk shape, and formed by hiving a central hole 17a and a pair of piercing holes 17b. Also, on the temperature sensor substrate 17, the temperature sensor 12 is implemented. The temperature sensor substrate 17 is attached to the stage bottom portion 10b by the tapping screws (not shown in the figures) piercing through the piercing holes 17b.

From the temperature sensor substrate 17, the lead wire (not shown in the figures) is drawn out rearward, and drawn around to the sensor substrate 13 through the gap Va.

The stage base mount 16 and the stage base 19 are formed by resin. The resin is a near infrared absorption grade of a black polycarbonate resin, for example. By forming the unit holder 15 with such metal as aluminum, a shielding effect is obtained, so that the influence of disturbance noises with respect to the sensor substrate 13 can be reduced.

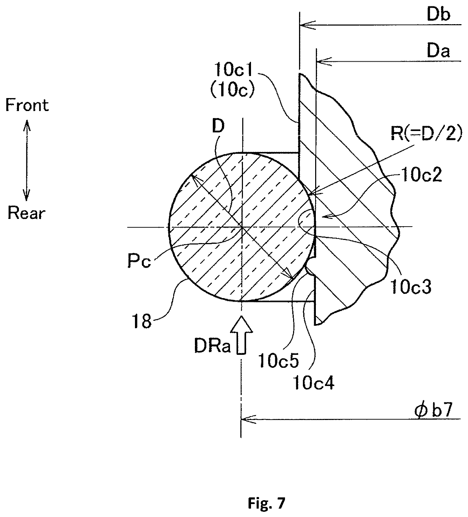

To the circumferential wall portion 10c of the stage base 10, a relay lens 18 is attached. Next, the relay lens 18 and its attachment method will be described with reference mainly to FIG. 7. FIG. 7 is an enlarged view of an SA section in FIG. 3.

In FIG. 7, on an outer circumferential surface 10c1 of the circumferential wall portion 10c of the stage base 10, a step unit 10c2 with a slightly larger diameter on a front side is formed around the entire circumference. The relay lens 18 is attached to this step portion 10c2.

The relay lens 18 is a ring shaped optical member with an inner diameter Da, and a cross sectional shape orthogonal to the extending direction is in a circular shape with a diameter D. A diameter $\phi b7$ of a central position Pc of a ring shaped portion is set to be equal to the diameter $\phi b$ (see FIG. 6) of the central position of the LED 14d implemented on the base substrate 14. The relay lens 18 is formed by a transparent polycarbonate resin having an optical transparency, for example.

The outer circumferential surface 10c1 of the circumferential wall portion 10c in the stage base 10 is formed such that an outer diameter on a front side than the step portion 10c2 has a diameter Db which is larger than the inner diameter Da of the relay lens 18. Then, at the step portion 10c2, it has a diameter that is gradually reduced by a concave curved surface 10c3 of a radius R (=D/2) as one goes rearward, and it is connected to a reduced diameter portion 10c4 having the same outer diameter as the inner diameter Da. Also, at the reduced diameter portion 10c4, a plurality of minute protrusions 10c5 are formed to be separated by a prescribed interval in the circumferential direction. The relay lens 18 is attached to be in tight contact with the concave curved surface 10c3 toward a front side, and the minute protrusions 10c5 are regulating a movement of the relay lens 18 to a rear side.

At a time of attaching the relay lens 18 to the step portion 10c2, the relay lens 18 is moved while undergoing the elastic deformation to widen the inner diameter from a rear side (see an arrow DRa), made to get overt the minute protrusions 10c5, and accommodated between the minute protrusions 10c5 and the concave curved surface 10c3.

As shown in FIG. 3, between the relay lens 18 and an inner surface 2b1 of the stage portion 2b in the lid body 2, a seal ring 19 is fitted by a strong fitting. The seal ring 19 is formed by a spring wire made of metal, for example. The seal ring 19 is pressing the relay lens 18 against the stage base 10 by the elastic repulsive force of the strong fitting, so that a position of the relay lens 18 is surely maintained without being displaced.

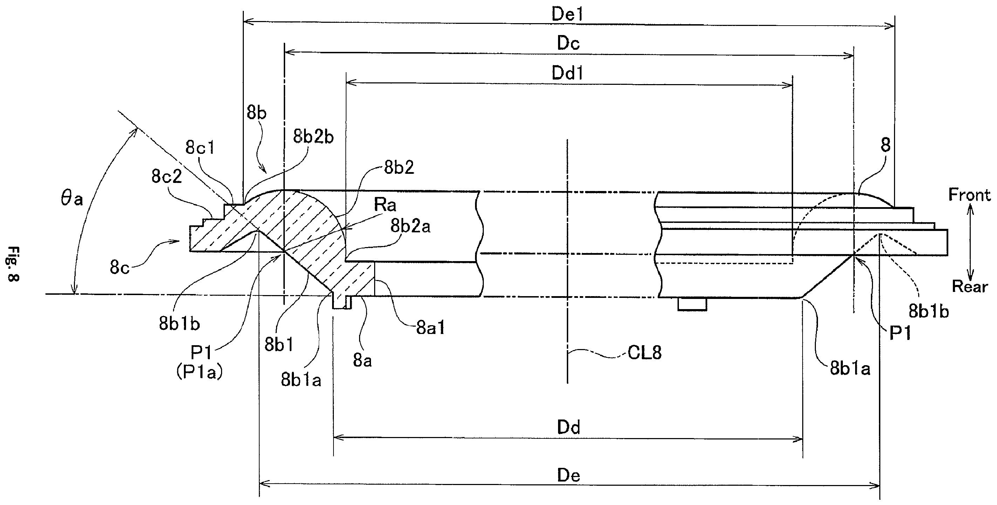

In front of the relay lens 18, the ring lens 8 already described is attached to be facing against the relay lens 18 in the front and rear direction. The ring lens 8 is formed by a transparent polycarbonate resin having an optical transparency, for example. FIG. 8 is a half cross sectional view (a cross section partially omitted) for explaining the ring lens 8.

As shown in FIG. 8, the ring lens 8 has a ring shaped base portion 8a formed in a ring shape and having a hole 8a1, a deflected portion 8b that is extending out by being inclined forward and outward in the diameter direction from the ring shaped base portion 8a, and a flange portion 8c that is projecting outward in the diameter direction from the deflected portion 8b. The ring shaped base portion 8a, the deflected portion 8b and the flange portion 8c are formed in a ring shape with an axial line CL8 extending from front to rear as the center.

The deflected portion 8b has a light incoming surface 8b1 on a rear side, and a light outgoing surface 8b2 on a front side. The light incoming surface 8b1 is a conical peripheral surface, passing through a virtual reference circle P1 with a diameter Dc, and inclined at an inclination angle $\theta a$ toward a front as it goes away from the axial line CL8. The light outgoing surface 8b2 is formed as a curved surface in which a cross sectional shape shown in FIG. 8 is extending in the circumferential direction with an arc shape in cross section with a radius Ra centered around a point P1a, when an intersection between a plane containing the axial line CL8 and the virtual reference circle P1 is set as the point P1a.

A range in the diameter direction in which the light incoming surface 8b1 is formed is such that an edge portion 8b1a on the inner diameter side has a diameter Dd that is smaller than the diameter Dc, and an edge portion 8b1b on the outer diameter side has a diameter De that is larger than the diameter Dc. A range in the diameter direction in which the light outgoing surface 8b2 is formed contains at least a range in the diameter direction in which the light incoming surface 8b1 is formed. More specifically, an edge portion 8b2a on the inner diameter side of the light outgoing surface 8b2 has a diameter Dd1 that is smaller than the diameter Dd, and an edge portion 8b2b on the outer diameter side has a diameter De1 that is larger than the diameter De.

The flange portion 8c has a ring shaped plane portion 8c1 that is connected to the edge portion 8b2b on the outer diameter side of the light outgoing surface 8b2 and orthogonal to the axial line CL8, and a ring shaped shelf portion 8c2 that is formed in a step shape with respect to the plane portion 8c1 and positioned on a rear side, on an outer side in the diameter direction of the plane portion 8c1.

As shown in FIG. 3, the ring lens 8 is attached to close a space between the peripheral portion of the front surface 10a of the stage base 10 and the inner surface 2b1 of the stage portion 2b of the lid body 2. In detail, the hole 8a1 of the ring shaped base portion 8a engages with the step portion formed on a periphery of the front surface 10a of the stage base 10, to seal with a mediating O-ring (not shown in the figure) and to be fixed by adhesive. The plane portion 8c1 of the flange portion 8c abuts to a rear surface of an inner flange 2b2 formed to be protruding inward at a tip end of the stage portion 2b, and the O-ring 82 is mediating between its rear surface and the shelf portion 8c2.

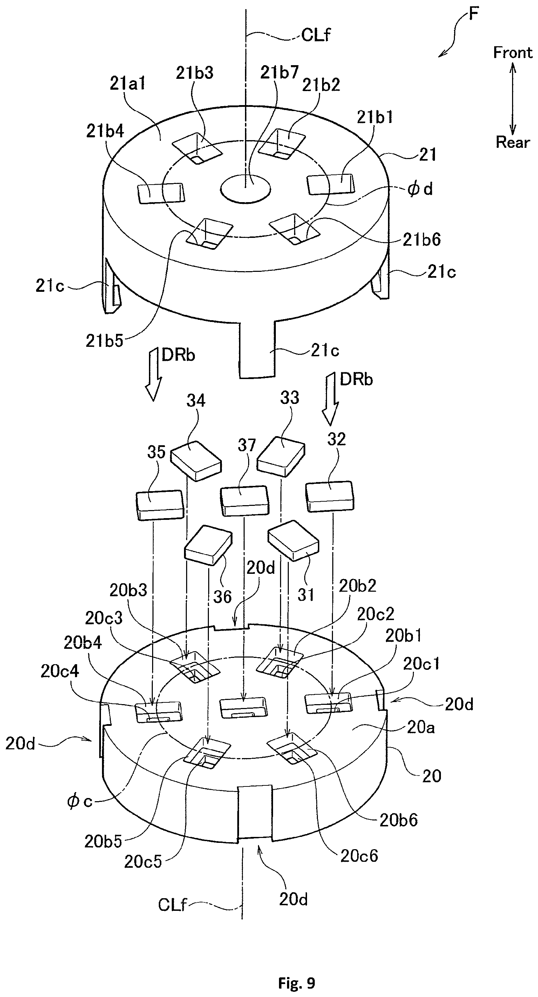

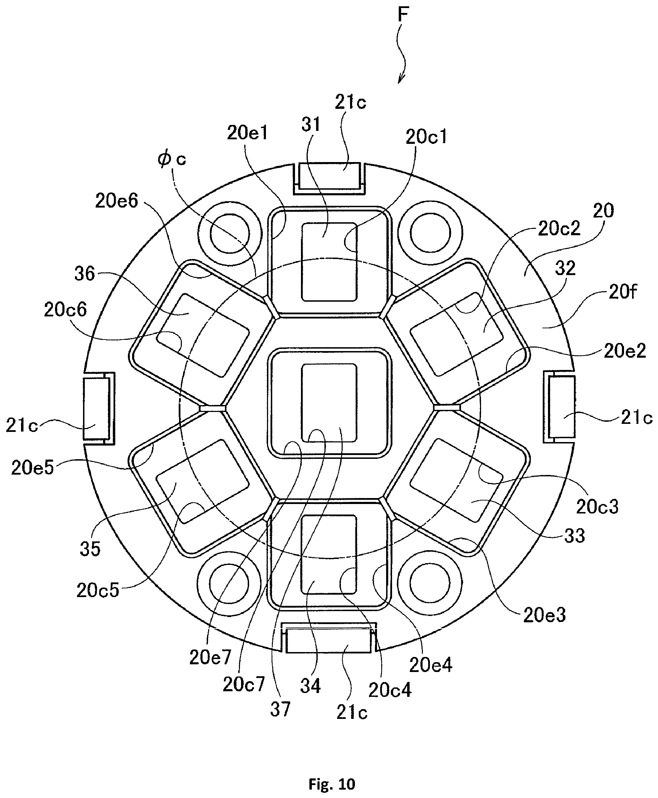

Next, the filter unit F will be described with reference to FIG. 9 and FIG. 10. The filter unit F is configured by having a disk shaped filter holder 20, a disk shaped holder cover 21 to be engaged with the filter holder 20 in a direction of an axial line CLf of the filter holder 20, and band-pass filters 31-37 that are a plurality (seven in this example) of optical band-pass filters to be sandwiched between the filter holder 20 and the holder cover 21. For seven band-pass filters 31-37, those having band-pass characteristics in which respective central wavelengths are different and having rectangular outer shapes are adopted.

The filter holder 20 and the holder cover 21 are formed by resin. The resin is a near infrared absorption grade of a black polycarbonate resin, for example.

For the wavelengths to measure the absorbance, m (where m is an integer greater than or equal to 2) types of wavelengths are selected, based on the absorbance wavelengths of the sugar. In this example, seven types of wavelengths $\lambda 1$-$\lambda 7$ with m=7 are selected. Also, as the photo sensors 13c, m sets of the photo sensors 13c1-13c7 are provided. Namely, the respective central wavelengths of the band-pass filters 31-37 are set to be wavelengths $\lambda 1$-$\lambda 7$ that are selected and set based on the conventionally known absorbance wavelengths of the sugar. Also, for the LEDs 14d, those having the emission central wavelengths corresponding to the central wavelengths $\lambda 1$-$\lambda 7$ respectively are selected. In detail, for the LEDs 14d, those having the emission central wavelengths that are equal to or close to the central wavelengths $\lambda 1$-$\lambda 7$ respectively are selected. The central wavelengths $\lambda 1$-$\lambda 7$ selected and set in this example are as follows. Also, the wavelengths in parentheses are the emission central wavelengths of the LEDs 14d selected and used in correspondence to the band-pass filters 31-37 respectively.

Band-pass filter 31 ... $\lambda 1$: 875 nm (880 nm)
Band-pass filter 32 ... $\lambda 2$: 900 nm (900 nm)
Band-pass filter 33 ... $\lambda 3$: 950 nm (950 nm)
Band-pass filter 34 . . . $\lambda 4$: 980 nm (980 nm)
Band-pass filter 35 ... $\lambda 5$: 1020 nm (1020 nm)
Band-pass filter 36 ... $\lambda 6$: 1050 nm (1064 nm)
Band-pass filter 37 ... $\lambda 7$: 1064 nm (1064 nm)

In the above noted example, the central wavelengths of the band-pass filters 32-35 and 37 other than the band-pass filters 31 and 36 are coinciding with the emission central wavelengths of the LEDs 14d used in correspondence respectively. Also, differences between the central wavelengths of the band-pass filters 31 and 36 and the emission central wavelengths of the corresponding LEDs 14d are 5 nm and 14 nm, respectively. With the wavelength difference of this level (less than or equal to 20 nm, for example), no considerable difference in the optical intensity for the respective central wavelengths of the band-pass filters will occur in the generally used LED emission spectrum. Because the emission spectrum is narrow for the general LED, it is preferable to select an LED having the emission central wavelength corresponding to the central wavelength of the band-pass filter. On the other hand, in the case of selecting an LED with a low electricity consumption for which the emission spectrum for high luminance is broad, it may not be necessary to select one having the emission central wavelength close to the central wavelength of the band-pass filter.

FIG. 9 is a schematic assembly diagram of the filter unit F. The filter holder 20 is formed in a disk shape centered around the axial line CLf, and six concave portions 21b1-20b6 formed at an angle pitch of 60° around the axial line CLf with a circle of a diameter φc centered around the axial line CLf as a central position and a concave portion 20b7 formed at a center are formed on a front surface 20a. The concave portions 20b1-20b7 are made to be rectangular depressions corresponding to the outer shapes of the band-pass filters 31-37.

The holder cover 21 has a base portion 21a formed in a disk shape, and four claw portions 21c extending out rearward from a peripheral portion of the base portion 21a and to be engaged with the filter holder 20. The claw portions 21c are arranged to be separated with each other by an angle pitch of 90° in the circumferential direction. On the base portion 21a, rectangular piercing holes 21b1-21b6 are formed at positions with an angle pitch of 60° centered around the axial line CLf and a circle of a diameter φd as a center in the diameter direction. Also, at the central position, a circular piercing hole 21b7 is formed.

The diameter φd is set equal to the diameter φc.

The holder cover 21 can be integrated with the filter holder 20, by approaching close to the filter holder 20 from a front side (see an arrow DRb) and engaging the claw portions 21c with engagement portions 20d provided on the filter holder 20. Namely, the filter unit F holding the band-pass filters 31-37 is formed by accommodating the respective band-pass filters 31-37 in the concave portions 20b1-20b7 of the filter holder 20, and engaging the holder cover 21 with the filter holder 20 from a front side.

FIG. 10 is a rear view of the filter unit F. On a rear surface 20f of the filter holder 20, pocket portions 20e1-20e7 with rectangular openings oriented to a front are formed at positions corresponding to the concave portions 20b1-20b7 formed on the front surface 20a. The concave portions 20b1-20b7 and the pocket portions 20e1-20e7 are coupled in the front and rear direction by the respective rectangular piercing holes 20c1-20c7. The central position in the diameter direction of the piercing holes 20c1-20c6 is on a circle with the diameter φc.

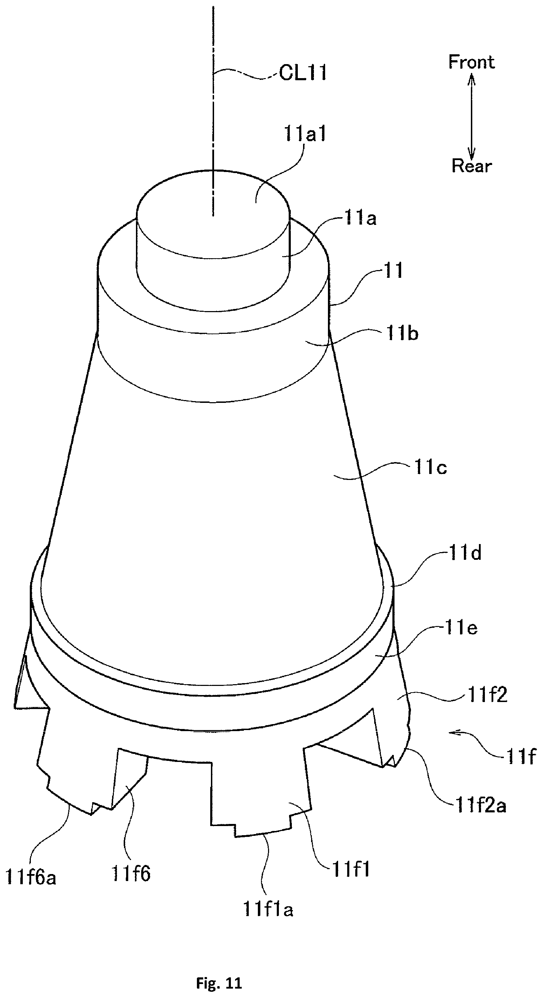

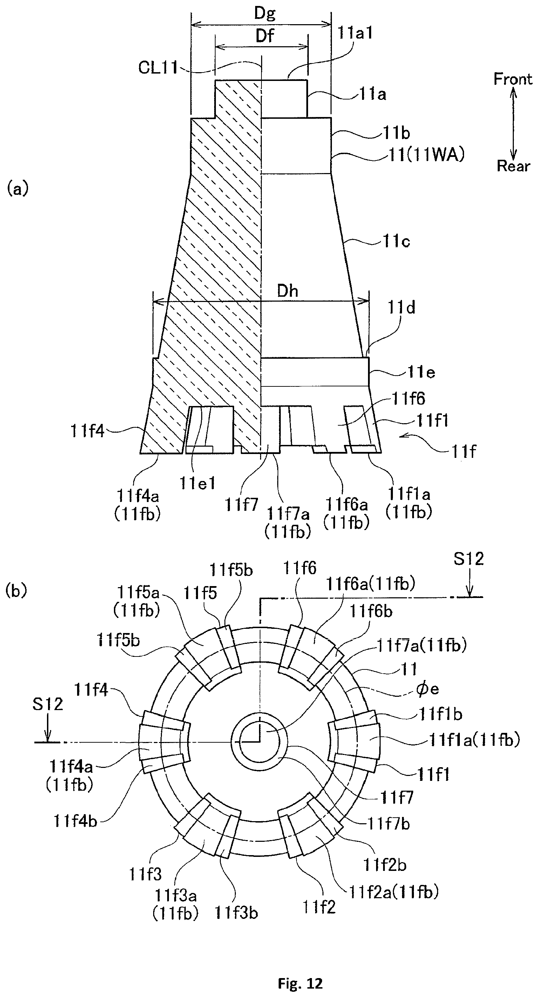

Next, the light guide member 11 will be described with references to FIG. 11 and FIG. 12. FIG. 11 is a perspective view looking at the light guide member 11 from an oblique front. FIG. 12 is a half cross sectional view (a) and a rear view (b) of the light guide member 11, wherein FIG. 12(a) is a half cross section at S12-S12 position in the rear view (b).

The light guide member 11 is formed as a transparent member having an optical transparency. A material is a transparent polycarbonate resin having an optical transparency, for example.

The light guide member 11 is formed to be long from front to rear. The light guide member 11 has a front end surface 11a1 as one end surface of its length, and is equipped with a front protruding portion 11a in a cylindrical shape with a diameter Df centered around an axial line CL11 extending from front to rear, an intermediate cylinder portion 11b in a cylindrical shape with a diameter Dg that is larger than the diameter Df, and a truncated cone portion 11c connected to a rear side of the intermediate cylinder portion 11b and having a gradually enlarging diameter as it goes toward a rear.

Moreover, the light guide member 11 is equipped with a rear cylinder portion 11e to be in a cylindrical shape with a diameter Dh via a step portion 11d that has an enlarged diameter in a direction orthogonal with respect to the axial line CL11, and a leg portion 11f having seven light guide protrusion portions 11f1-11f7 formed to be protruding independently toward a rear, from a rear surface 11e1 of the rear cylinder portion 11e.

Six light guide protrusion portions 11f1-11f6 are formed on a circle with a diameter φe, with an equal angle interval (at an angle pitch of 60°) centered around the axial line CL11. The remaining one light guide protrusion portion 11f7 is formed in a column shape at a central position.

Also, the light guide protrusion portions 11f1-11f6 have engagement portions 11f1a-11f6a protruding to a rear further with a reduced diameter in stepped portions 11f1b-11f6b at rear tip end portions. Also, the light guide protrusion portion 11f7 has an engagement portion 11f7a protruding to a rear further with a reduced diameter in a stepped portion 11f7b at a rear tip end portion. The positions of the stepped portions 11f1b-11f7b of the engagement portions 11f1a-11f7a and the front and rear direction positions of the tip end portions are set to be the same positions with each other, respectively.

The engagement portions 11f1a-11f7a have the stepped portions abutted to the front surface of the base portion 21a of the holder cover 21, and made to enter into the piercing holes 21b1-21b7 of the holder cover 21 in the filter unit F from a front side.

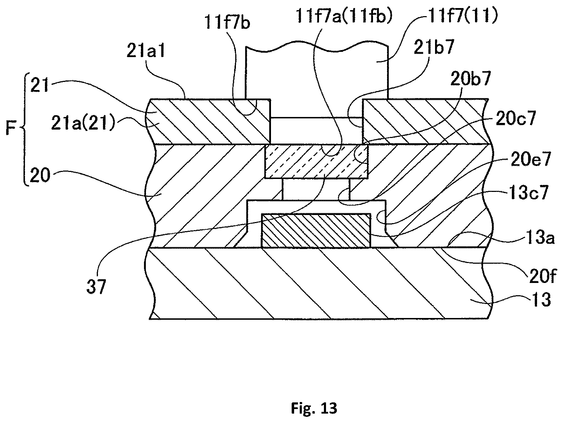

The attachment state of the filter unit F and the light guide member 11 with respect to the sensor substrate 13 will be described hereunder with reference to FIG. 13. This attachment is so done, as described above, that the filter unit F and the light guide member 11 are sandwiched in the front and rear direction, between the stage base mount 16 and the sensor substrate 13, by fixing the stage base mount 16 with the tapping screws Na.

FIG. 13 is a schematic cross sectional view of a SB section in FIG. 3. Namely, it shows the assembled state of the photo sensor 13c7 of the sensor substrate 13, the filter unit F, and the light guide protrusion unit 11f7 in the light guide member 11. It is similar for other photo sensors 13c1-13c6, and it will be described as a representative example.

As shown in FIG. 13, to the front surface 13a of the sensor substrate 13, the rear surface 20f of the filter holder 20 is abutted. The photo sensor 13c7 implemented on the front surface 13a is entering into the pocket portion 20e7 formed on the filter holder 20 of the filter unit F. In the concave portion 20b7 of the filter holder 20, the band-pass filter 37 is inserted. The movement of the band-pass filter 37 toward a front is regulated by being pressed by the piercing hole 21b7 of the holder cover 21 that is formed to be smaller than the outer shape of the band-pass filter 37. The band-pass filter 37 is in an opposing position in front of the photo sensor 13c7.

In the piercing hole 21b7 of the holder cover 21, the engagement portion 11f7a of the light guide protrusion portion 11f7 of the light guide member 11 is entered and engaged from a front side. The stepped portion 11f7b of the light guide protrusion portion 11f7 is abutted to the front surface 21a1 of the base portion 21a of the holder cover 21.

The operation of the saccharinity meter 51 with the above explained configuration is controlled by the control unit CT. FIG. 14 is a diagram for explaining a configuration of a control system in the saccharinity meter 51. The control unit CT has a central processing unit (CPU) CT1, a correction unit CT2, a light intensity processing unit CT3, a display control unit CP4, a light amount control unit CT5 for controlling light amount emitted from the LED group 14dG, and a memory unit CT6.

A length L and a width W (see FIG. 2) and a thickness H (see FIG. 3) defining the outer dimensions of the saccharinity meter 51, are set roughly as follows, for example.
L=113 mm, W=63 mm, H=43 mm

Also, the outer diameter φf (see FIG. 3) of the measurement portion K1 is set to be 48 mm, for example.

Next, the operation of the saccharinity meter 51 with the above described configuration will be described. First, the operator brings the measurement portion K1 of the saccharinity meter 51 into contact with the fruit or vegetable AS that is a measurement target. More specifically, in the case of measuring the fruit or vegetable AS after the harvest, for example, the operator can make the measurement by mounting the saccharinity meter 51 on a mount such as a table 91, in such an orientation that the outer abutting portion 7 and the inner abutting portion 9 of the measurement portion K1 become an upper end, and mounting the target fruit or vegetable AS on the measurement portion K1, as shown in FIG. 15. In the case of measuring the fruit or vegetable AS with a depression in this measurement manner, it is advisable to mount a portion without a depression or a portion with less depression on the measurement portion K1.

In the case of measuring the fruit or vegetable AS that is before the harvest and still growing, or the fruit or vegetable AS that is heavy or large, the operator holds the grip portion K2 and makes the measurement by bringing the measurement portion K1 into contact with a surface of the fruit or vegetable AS, as shown in FIG. 16. The measurement portion K1 is formed to be protruding with respect to the grip portion K2. By means of this, when the saccharinity meter 51 is mounted on a mount and the fruit or vegetable AS is mounted on the measurement portion K1, the convex portions of the uneven surface of the fruit or vegetable AS will abut to the grip portion K2, so that the fruit or vegetable AS will not be mounted unstably. Also, in the case of conducting measurement by holding the saccharinity meter 51 and abutting the measurement portion K1 to the fruit or vegetable AS by the operator, abutment of the holding fingers of the operator to the fruit or vegetable AS is effectively prevented from occurring. For this reason, there is no possibility for the measurement precision to be lowered as a gap between the outer abutting portion 7 and the inner abutting portion 9 and the fruit or vegetable AS is not generated and an obstacle external light does not enter.

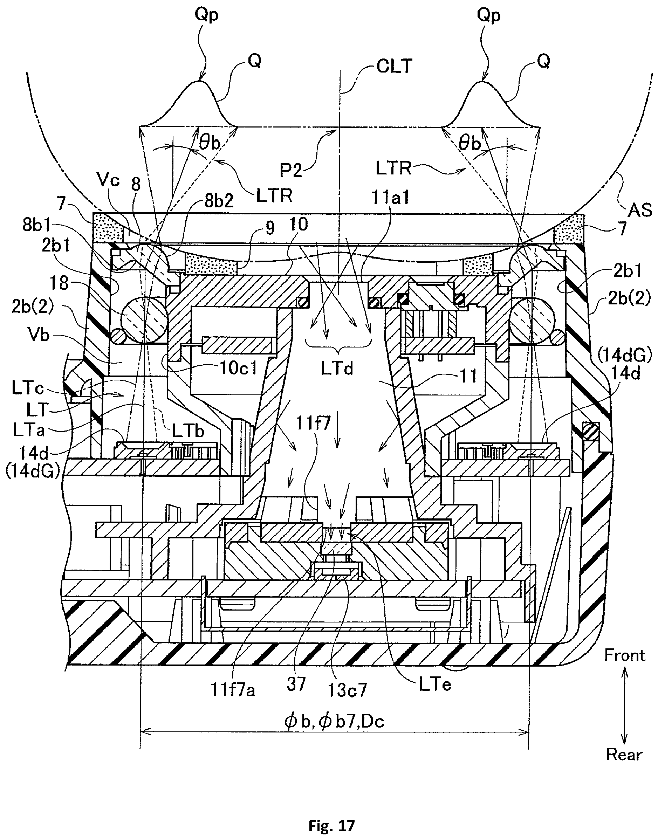

Next, the specific measurement operation of the saccharinity meter 51 will be described with references mainly to FIG. 14 and FIG. 17. FIG. 17 is a schematic diagram for explaining optical paths at a time of making the sugar content measurement by the saccharinity meter 51, which utilizes a portion of the measurement portion K1 of FIG. 3. In FIG. 17, the shading is not depicted for the light guide member 11 that is shown in cross section, so as not to make the drawings too cumbersome or complicated.

First, the diameter $\phi b$ (see also FIG. 6) at which the LEDs 14d are arranged to be separated in the circumferential direction, the diameter $\phi b7$ (see FIG. 7) at a center of the relay lens 18, and the diameter Dc (see FIG. 8) of the virtual reference circle P1 of the ring lens 8 are set to be equal. The diameter $\phi b$ (=$\phi b7$=Dc) is set to be a diameter of 38 mm, for example.

(1) After turning on of the power of the saccharinity meter 51, the user or operator mount the fruit or vegetable AS to be measured on the outer abutting portion 7 and the inner abutting portion 9 of the measurement portion K1 of the saccharinity meter 51, or the measurement portion K1 of the saccharinity meter 51 is pressed to be in tight contact with the fruit or vegetable AS to be measured (fruit or vegetable contacting step).
The fruit or vegetable AS is a fruit or a vegetable which may be tomato, apple, watermelon, and the like. The outer abutting portion 7 and the inner abutting portion 9 are roughly in tight contact with the surface of the fruit or vegetable AS while being compressed, due to the own weight of the fruit or vegetable AS or the pressing force of the user.
(2) The user presses the press button 6, to turn the switch 13d to the ON state. A signal indicating that it is turned to the ON state is sent out from the switch 13s to the central processing unit CT1 of the control unit CT.
(3) When the central processing unit CT1 comprehends that the switch 13d is turned to the ON state, it outputs commands to the light amount control unit CT5 to cause the LED group 14dG to emit lights (light emitting step). The light amount control unit CT5 causes the LED group 14dG to emit light in such manner that light LT having at least two different light amount is emitted (multiple light emitting step). More specifically, after the light amount control unit CT5 causes the LED group 14dG to emit light LT having a first amount of light, it causes the LED group 14dG to emit light LT having a second amount of light which is different from the first amount of light, and if necessary, it causes the LED group 114dG to emit light LT having a third amount of light which is still further different from both the first and second amount of light.

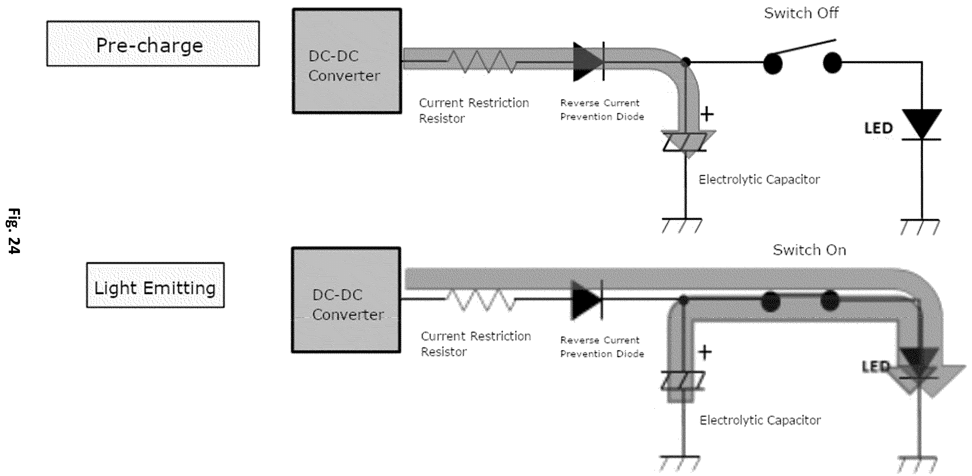

Increase of driving currents due to increase of light amount (high-brightness) of LED group 14dG and increase of forward voltage (VF) of LEDs require the power source to supply greater electric power than that of the conventional one. To cope with this requirement, in the saccharinity meter 51 of this invention, it is so arranged that power is supplied to the LED group 14dG after electric charge is once pre-charged in an electrolytic capacitor, for example. Here, if a current restricting resistor is not provided, an electric charge cannot be supplied to the electrolytic capacitor because an error occurs caused by increase of load on battery. An electric charge is supplied to the electrolytic capacitor through the current restricting resistor. This necessitates time for charging the electrolytic capacitor, thereby the measurement time required becomes long. In the meter of this invention, as shown in FIG. 24, the current restriction resistor restricts charge currents to the electrolytic capacitor, while, at the timing of light emitting state, LED group 14dG is supplied with currents from both the electrolytic capacitor and a power source (a DC/DC converter) under the condition that the current from the electrolytic capacitor is effectively restricted by the current restriction resistor. According to the above construction, such effects that time required for measurement is shortened, that capacitance of the electrolytic capacitor can be reduced, and that error operation caused by increase in battery load can be prevented from occurring, can be achieved.

Additionally, in order to achieve higher brightness light emission of LED group 14dG, it is effective to change the emission time of LED group 14dG. For example, high brightness can be achieved by making the light emitting time to be 2.5 ms and by increasing the current (for example, 300 mA).

The light LT emitted upward from each LED 14d of the LED group 14dG passes through the relay lens 18 and reaches the ring lens 8. A space Vb to be a path of the light LT from the LED 14d to the ring lens 8 has a diameter direction outer side that is closed by the inner surface 2b1 of the stage portion 2b of the lid body 2, and a diameter direction inner side that is closed by the outer circumferential surface 10c1 of the stage base mount 16 and the stage base 10. As clearly understood, the space Vb is made to be a space in which the diameter direction side is closed while only the axial direction side is open. A tip end portion on the axial direction side is closed by the ring lens 8. Accordingly, the emitted light from the LED group 14dG will not reach to the fruit or vegetable AS without passing through the ring lens 8.

In FIG. 17, the main optical axis LTa of the light LT emitted from the LED 14d is indicated by a solid line. When the main optical axis LTa passes through a center of the relay lens 18 and reaches the light incoming surface 8b1 of the ring lens 8, because the light incoming surface 8b1 is inclined forward as it goes to the diameter direction outer side as shown in FIG. 8, the main optical axis LTa is emitted forward from the light outgoing surface 8b2 at an angle θb of the emitted light according to that inclination angle θa and the refractive index of the material of the ring lens 8.

It is preferable to set the angle θb of the emitted light to be 0<θb<45°. It is preferable to set the angle θb of the emitted light to be large in accordance with increase in the diameter Dc of the virtual reference circle P1 of the ring lens 8. By so setting, it becomes easier for the lights LTR (to be described below) entered into the fruit or vegetable AS to be concentrated at a center in the interior of the fruit or vegetable AS. In the case where the diameter Dc is approximately 40 mm as in the saccharinity meter 51, it is suitable for the angle θb to be about 20°.

The lights emitted forward from this light outgoing surface 8b2 become ring shaped lights LTR having a width in the in- and out- direction of the diameter and containing the main optical axis LTa. Namely, in FIG. 17, among the ring shaped lights emitted from the light outgoing surface 8b2, a light path LTb from the LED 14d of the light emitted to be most deflected to the inner diameter side from the ring lens 8 is indicated by a dashed line. Also, a light path LTc from the LED 14d of the light emitted to be most deflected to the outer diameter side is indicated by a one dot chain line.

Moreover, in FIG. 17, the intensity characteristics Q of the ring shaped light LTR at the front and rear direction position P2 after the emission are shown. From these, the intensity characteristic Q in the diameter direction of the ring shaped lights LTR emitted from the ring lens 8 is a characteristic having a peak Qp that abruptly rises at the main optical axis LTa, in which the intensity rapidly decreases as it goes toward the inner diameter side and the outer diameter side.

On the other hand, the intensity characteristic in the circumferential direction of the ring shaped lights LTR is almost constant. Namely, the intensity characteristic in the circumferential direction of the ring shaped lights LTR is such that, even though the plurality of LEDs 14d are arranged to be separated in the circumferential direction, it is averaged to be almost the same level in the case where the plurality of LEDs 14d are turned on simultaneously, due to the emission characteristic of each LED 14d that is spread to the circumferential direction as well and the slight internal diffusion occurring in the relay lens 18 and the ring lens 8.

The ring shaped lights LTR emitted from the ring lens 8 have the main optical axis LTa that is deflected at the angle θb in the direction of approaching to the light receiving axial line CLT as described above (the light receiving axial line CLT is coinciding with the axial line of the light guide member 11). In other words, the ring shaped lights LTR emitted from the ring lens 8 will be propagated to have the reduced diameter after emitted from the ring lens 8.

At the central portion on the inner side of the inner abutting portion 9, the front end surface 11a1 of the light guide member 11 is exposed.

(4) The lights LTR emitted from the ring lens 8 are irradiated in a ring shape onto the surface of the fruit or vegetable AS, and enter into the interior of the fruit or vegetable AS.

(5) The lights LTR entered into the interior of the fruit or vegetable AS are irregularly reflected in the interior while absorbed by the characteristic corresponding to a state of the fruit or vegetable AS, and a part of them is emitted to the external.

(6) The part of the light emitted to the external enters into the interior of the light guide member 11 from the front end surface 11a1 of the light guide member 11 that is exposed to the external. The lights entered into the interior of the light guide member 11 are those lights that are emitted from the LED group 14dG, passed through the interior of the fruit or vegetable AS, and returned, so that they are referred to as return lights LTd in the following. The return lights LTd are propagated through the interior of the light guide member 11, and guided to the light guide protrusion portions 11f1-11f7 of the leg portion 11f.

In this way, the return lights LTd are those lights that are propagated in a ring shape through the interior of the fruit or vegetable AS, reflected in the interior, and emitted to the external from the central portion with respect to the ring shaped entering portion at which they are entered. For this reason, even if there is a bias in the absorbance at each portion in the internal texture of the fruit or vegetable AS, they are going to be lights in which the bias is averaged.

Also, the lights LTR injected into the fruit or vegetable AS are in the ring shape, and deflected in the direction of approaching to the light receiving axial line CLT. For this reason, among the lights irregularly reflected in the interior of the fruit or vegetable AS, a ratio of the lights injected into the front end surface 11a1 at the central lower portion becomes high compared with the case of not deflected (the angle θb=0).

As a result, in the saccharinity meter 51, the return lights LTd are obtained at high efficiency with respect to the emitted lights of the LEDs 14d, so that even if there is a bias in the absorbance of the internal texture of the fruit or vegetable AS, they can be obtained as lights that are hard to be affected by that bias and reflecting the absorbance of the fruit or vegetable AS in higher precision.

(7) The return lights LTd guided to the light guide protrusion portions 11f1-11f7 of the leg portion 11f are uniform lights without the bias in the characteristics with each other, and they are emitted from the respective rear end surfaces 11fb of the respective engagement portions 11f1a-11f7a toward the band-pass filters 31-37, as the protrusion portion emitted lights LTe (LTe1-LTe7). The rear end surface 11fb is the other rear end surface with respect to the front end surface 11a1 that is one end surface of its length in the light guide member 11.

(8) The protrusion portion emitted lights LTe emitted from the engagement portions 11f1a-11f7a are spectrally dispersed according to the respective spectral characteristics by the band-pass filters 31-37 and are received by the photo sensors 13c1-13c7 (light receiving step).

(9) The photo sensors 13c1-13c7 detect the intensities Q1-Q7 that are the respective received light intensities, and forward the intensity information to the light intensity processing unit CT3 (see FIG. 14). Namely, the intensities Q1-Q7 obtained from the photo sensors 13c1-13c7 indicate the spectral intensities at the central wavelengths $\lambda 1$-$\lambda 7$ of the band-pass filters 31-37, respectively.

(10) The light intensity processing unit CT3 obtains the absorbance of the wavelengths $\lambda 1$-$\lambda 7$ respectively by the known calculation method from the intensities Q1-Q7, and calculates the Brix value Y from each absorbance (light intensity processing step). The exemplary concrete calculation method is as follows.

In general, the absorbance A of a wavelength $\lambda$ is given by the equation (1), where IO ($\lambda$) is the intensity of light of a wavelength $\lambda$ entering into the measurement target to be a reference, IS ($\lambda$) is the intensity of light of a wavelength $\lambda$ emitted from the measurement target.

$$A = \log [\, IO\,(\lambda)\,/\,IS\,(\lambda)\,] = \log IO\,(\lambda)\cdot\log IS\,(\lambda)\ldots\ldots\ldots(1)$$

Among seven types of wavelengths $\lambda 1$-$\lambda 7$, the wavelength $\lambda 6$ is taken as a wavelength to be a reference, the absorbances A1-A5, A7 for the other six types of wavelengths $\lambda 1$-$\lambda 5$, $\lambda 7$ respectively are obtained by the following equations (2-1)-(2-6).

$$A_1 = A(\lambda_1) - A(\lambda_6) = \log\frac{I_0(\lambda_1)}{I_s(\lambda_1)} - \log\frac{I_0(\lambda_6)}{I_s(\lambda_6)} \quad \cdots \quad (2\text{-}1)$$

$$A_2 = A(\lambda_2) - A(\lambda_6) = \log\frac{I_0(\lambda_2)}{I_s(\lambda_2)} - \log\frac{I_0(\lambda_6)}{I_s(\lambda_6)} \quad \cdots \quad (2\text{-}2)$$

$$\cdots$$

$$A_6 = A(\lambda_7) - A(\lambda_6) = \log\frac{I_0(\lambda_7)}{I_s(\lambda_7)} - \log\frac{I_0(\lambda_6)}{I_s(\lambda_6)} \quad \cdots \quad (2\text{-}6)$$

Based on these equations, the Brix value Y is calculated by the following equation (3).

$$Y = PL0 + A_1 \times PL1 + A_2 \times PL2 + A_3 \times PL3 + A_4 \times PL4 + A_5 \times PL5 + A_6 \times PL6 + T_1 \times PL7 + T_2 \times PL8$$

$$(3)$$

Here, PL0-PL8 are coefficients obtained in advance by the multiple regression analysis using the absorbance data for a plurality of measurement targets (fruit or vegetable AS). Also, the temperature T1 is a surface temperature of the measurement target (fruit or vegetable AS) measured by the temperature sensor 12, and the temperature T2 is a temperature corresponding to the casing K measured by the temperature sensor 12.

The light intensity processing unit CT3, according to changes in light intensity emitted from the LED group 14dG, obtains respective absorbencies of wavelengths λ1-λ7 and, thereafter calculates the Brix value Y from each absorbance. By mapping the light intensity emitted from the LED group 14dG to the depth of the fruit or vegetable AS, the distribution of Brix value Y in the depth direction of the fruit or vegetable AS is obtained.

Also, the light intensity processing unit CT3 can obtain a calibration curve (standard curve) of sugar content of the fruit or vegetable AS, based on light absorbencies of at least two intensities of lights and the Brix value of at the corresponding light intensities in the depth direction of the fruit or vegetable AS.

As an example, the method and flow for obtaining the calibration curve of sugar content of watermelon as AS (fruit or vegetable) by evenly dividing the watermelon into four in the direction of depth taken into consideration the fact of sugar content differences between the inner portion and the outer portion of the watermelon will be explained hereunder.

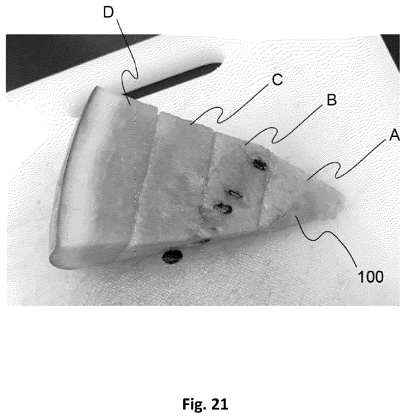

FIG. 21 shows a piece of watermelon 100 in which four potions from the center to skin of the watermelon are shown by A-D. For the watermelon 100 shown in FIG. 21, Brix values of portions A-D are measured at a plurality of points at each portion and then are averaged. Thus obtained averaged values are given in the following TABLE 1. In TABLE 1, the upper portion corresponds to the top portion of the watermelon 100 before cutting, the middle portion corresponds to the peripheral portion of the watermelon 100 before cutting, and the lower portion corresponds to the bottom portion of the watermelon 100 before cutting.

|  | Center Side ⇔ Skin Side | | | |
|  | A | B | C | D |
| Upper Portion | 11.7 | 11.7 | 10.7 | 7.9 |
| Middle Portion | 11.5 | 11.4 | 10.5 | 7.7 |
| Lower Portion | 11.4 | 11.4 | 10.4 | 7.7 |

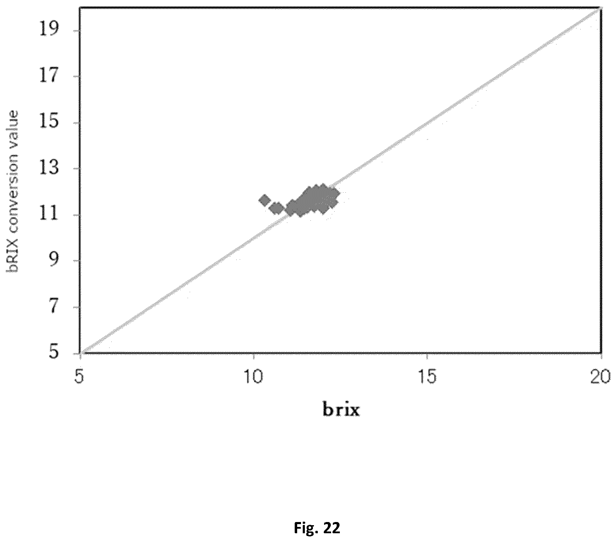

As understood from TABLE 1, the sugar content (Brix value) is substantially the same where the depth in the depth direction is constant, but it abruptly decreases at the skin portion (portion D). From the sugar content information where the depth is constant, obtainable sugar content range from one watermelon 100 is small. Where the light intensity from the LED group 14dG is constant, namely, the depth in the depth direction is constant, the Brix values are measured and multiple regression analysis is performed thereon. Results are shown in FIG. 22. Multiple determination coefficient R2 is 0.3 and it shows that the Brix values are concentrated at a certain specific portion.

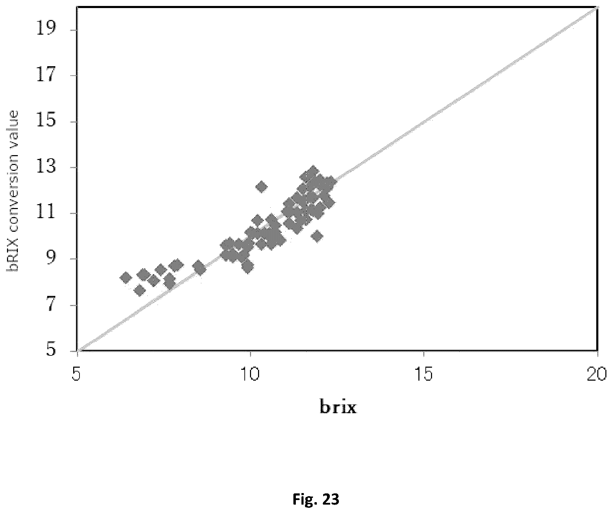

On the other hand, where the multiple regression analysis is performed in combination of the light intensity emitted from LED group 14dG and the sugar content information in the depth direction, the results are shown in FIG. 23. Here, the combination of the light intensities of LED group 14dG and the corresponding depth are as follows:

Luminus electric current: 50mA       Depth: 2 to 4 cm
Luminus electric current: 250mA      Depth: 6 to 8 cm

As result of performing the multiple regression analysis on the Brix values of the light intensity data and the Brix values at the respective depth obtained under the above conditions, the multiple determination coefficient R2 is 0.79. As explained above, by changing in light intensity and utilizing the sugar content information in the depth direction, sufficient sugar content range and precise calibration curve can be obtained on the fruit or vegetable which has sugar content distribution between the inner portion and outer portion thereof.

As shown in FIG. 14, the control unit CT has a correction unit CT2. The correction unit CT2 makes the closed control of the light amount of the LED group 14dG, based on the light amount information JL from the FB photo sensor group 14eG and the temperature information JT from the temperature sensor 12.

As the general characteristics of the LED, the emitted light amount therefrom changes as the temperature increases. It applies also to the LEDs 14d. The saccharinity meter 51 has the FB photo sensor group 14eG, the temperature sensor 12, and the correction unit CT2, so that the emitted light amount of the plurality of LEDs 14d can be made constant while changes in the emitted light amount with respect to time lapse are well stabilized and suppressed. By means of this, the measurement precision of the saccharinity meter 51 is further improved. The control unit CT is not limited to that which controls to cause all of the LEDs 14d of the LED group 14dG to emit lights simultaneously. The control unit CT may cause the corresponding LEDs 14d to emit lights sequentially in time sequence, for each of six types of wavelengths that are set, or in an order of the arrangement in the circumferential direction, and measure the received light intensities of the photo sensors 13c1-13c7 in each occasion. When the LED group 14dG is caused to emit lights simultaneously, the electricity consumption becomes large even in a short period of time, so that in the case where the reduction of the load on the power source is necessary, the latter method of causing the plurality of LEDs 14d to emit lights sequentially in time series and measuring is preferable.

The saccharinity meter 51 described above that is the nondestructive measurement apparatus for the fruit or vegetable uses the plurality of LEDs 14d as the light source of lights to be irradiated onto the fruit or vegetable AS. By means of this, the saccharinity meter 51 requires less electricity consumption for the light source and less installment space, and can be made so compact that the casing K can be held by one hand. Therefore, the measurement can be made easily even for the fruit or vegetable before the harvest that is still growing.

The saccharinity meter 51 has the plurality of LEDs 14d arranged in the circumferential direction, and the emitted light from each LED 14d is made to be irradiated toward the fruit or vegetable AS through the ring shaped ring lens 8. For this reason, the lights are made to be irradiated and injected as a ring shaped light bundle with respect to the fruit or vegetable AS. By means of this, even if there is a bias in the absorbance of the internal texture of the fruit or vegetable AS, the averaged emitted lights that are hard to be affected by that bias can be obtained, and the measurement result that is well reflecting a state of the fruit or vegetable AS can be obtained.

The ring lens 8 is given with the optical characteristic in which the injected light from the LED 14d is deflected toward a center of the ring lens 8 and emitted. For this reason, the intensities of the return lights that are reflected in the interior of the fruit or vegetable AS and emitted toward a central portion of the ring lens 8 can be obtained to be high, so that the utilization efficiency of the emitted lights of the LEDs 14d is high. By means of this, the saccharinity meter 51 is suitable for a handy type which is capable of being made to have a low electricity consumption, and which is driven by a battery, by suppressing the emission light intensity for the fruit or vegetable with a high optical transparency and the like. Also, the return lights sufficient for the measurement can be easily obtained even for the fruit or vegetable with a low optical transparency due to a thick skin and the like, so that the saccharinity meter 51 has many types of the fruits and vegetables that can be measured and it is superior in versatility.

The saccharinity meter 51 has the ring shaped relay lens 18 for concentrating the lights from the LEDs 14d to the ring lens 8, in a middle of the emitted light path from the LEDs 14d to the ring lens 8. As the relay lens 18 is arranged, the emitted lights of the LEDs 14d can be introduced to the ring lens 8 and irradiated onto the fruit or vegetable AS at higher efficiency. Also by means of this, the saccharinity meter 51 becomes capable of being made to have a low electricity consumption, so that it is suitable for the handy type. Also, the return lights sufficient for the measurement can be obtained more easily even for the fruit or vegetable with a low optical transparency due to a thick skin and the like, so that the saccharinity meter 51 has many types of the fruits and vegetables that can be measured and it is superior in versatility.

Also, as the relay lens 18 is arranged, the light path distance between the LEDs 14d and the ring lens 8 can be made longer. For this reason, the ring lens 8 can be arranged at a position closer with respect to the fruit or vegetable AS. By means of this, the emitted light bundle from the ring lens 8 can be irradiated and injected onto the fruit or vegetable AS in a sufficiently narrow ring shape, so that the injected light intensity per unit area can be made higher. Consequently, the utilization efficiency of the emitted lights from the LEDs 14d is further improved. Also, the influence due to the injected light from the external can be suppressed to a substantially ignorable level. By means of this, the saccharinity meter 51 can carry out the measurement in high precision.

Also, as the length or distance of the light path between the LEDs 14d and the ring lens 8 can be made longer by arranging the relay lens 18, the stage portion on which the fruit or vegetable AS is mounted can be made to protrude sufficiently, with respect to the grip portion which is held by hand. By means of this, as shown in FIG. 16, even in the case where the fruit or vegetable AS is measured by holding the grip portion K2, the fruit or vegetable AS and the fingers holding the grip portion K2 will not be abutted to each other, so that the measuring operation can be carried out in good feel, easily, and at high efficiency. Also, when a slight displacement occurs in the implementation positions of the LEDs 14d on the base substrate 14 due to the unevenness in the manufacturing, a slight difference also occurs in the emitted light angle of each LED 14d. Also, in the case of using the packaged compound LEDs such as the 3-wavelength compound LEDs described above, the emitted light position is slightly different in the diameter direction for each emitted light central wavelength. In contrast, the saccharinity meter 51 has the relay lens 18 as a reduction optical system lens between the LEDs 14d and the ring lens 8. Namely, the relay lens 18 produces a reduction system of the light source from the lights emitted from the ring shaped light source (the plurality of LEDs 14d arranged in a ring shape), and injects light into the ring lens 8. Then the ring lens 8 functions to irradiate the injected reduction system of the light source onto the fruit or vegetable as ring shaped light beams. For this reason, even if the displacement occurs in the emitted light angle, in conjunction with the slight displacement in the implementation positions of the LEDs 14d, or the slight displacement in the diameter direction of the emitted light positions in the case where the LEDs 14d are the compound LEDs, an influence affecting the measurement caused by the above displacement is small or minimum. For this reason, high precision measurement can be achieved by the saccharinity meter 51 of this invention.

The saccharinity meter 51 has the inner abutting portion 9 and the outer abutting portion 7 respectively in the inner and outer positions in the diameter direction with respect to the ring lens 8. By means of this, as shown in FIG. 17, a closed space Vc is formed by the ring lens 8, the fruit or vegetable AS, the inner abutting portion 9 and the outer abutting portion 7, in a state in which the fruit or vegetable AS is provided at the measurement portion K1.

Namely, on the outer diameter side with respect to the ring lens 8, a space between the stage portion 2b and the fruit or vegetable AS is closed by the outer abutting portion 7. Also, on the inner diameter side, a space between the stage portion 2b and the fruit or vegetable AS is closed by the inner abutting portion 9. By means of this, the ring shaped lights LTR emitted from the ring lens 8 will not reach the front end surface 11a1 of the light guide member 11, and also will not leak to the external of the diameter outer side. Consequently, the return lights LTd offering to the measurement in the saccharinity meter 51 are necessarily going to be lights incoming from the fruit or vegetable AS, so that the measurement precision is improved. Also, as the lights LTR do not leak externally, the utilization efficiency of the emitted lights of the LEDs 14d is improved.

The saccharinity meter 51 is equipped with the light guide member 11 with its length in the light receiving axial line CLT direction, and one end side of the length of the light guide member 11 is made to be the front end surface 11a1 of the light incoming surface for the return lights LTd, and another end side is made to be the light outgoing surface for the return lights LTd toward the photo sensors 13c. By means of this, the return lights LTd entered from the light incoming surface are guided to the light outgoing surface for a relatively long distance in conjunction with the inner surface reflection of the light guide member 11, so that the lights reached to the light outgoing surface become uniform lights regardless of positions reached on the light outgoing surfaces. Consequently, the saccharinity meter 51 has no bias in the characteristics of lights incoming to the photo sensors 13c1-13c7 respectively, so that the measurement can be carried out at high precision.

With this configuration, the photo sensors 13c1-13c7 are arranged on a rear side, i.e., at farther positions from the outer abutting portion 7 and the inner abutting portion 9, than the LED group 14dG to be the light source, in the positional relationship in the front and rear direction within the casing K. Consequently, the sensor substrate 13 on which the photo sensors 13c1-13c7 are mounted is arranged in vicinity of the rear surface 1a of the box body 1, and the light guide member 11 is arranged so as to penetrate through the hole 14g of the base substrate 14.

Next, the calibration of the saccharinity meter 51 will be described. It is desired for the saccharinity meter 51 to carry out the calibration periodically or regularly, in order to maintain the measurement precision of the saccharinity meter 51 and to, more surely, obtain a consistency with the measurement results of other machines and of the past.

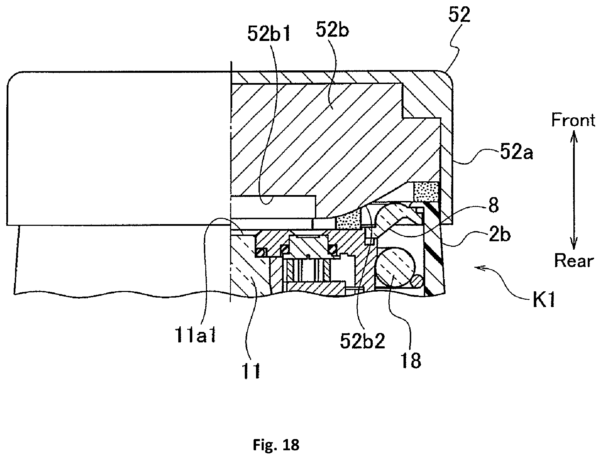

In the saccharinity meter 51, a standard lid body 52 for the purpose of the above calibration is prepared. FIG. 18 is a half cross sectional view for explaining a state of use of the standard lid body 52, where the standard lid body 52 and a part of the measurement portion K1 in the casing K are shown.

The standard lid body 52 has a column shape covering a tip end opening of the measurement portion K1, and it is used by putting on the stage portion 2b of the lid body 2. The standard lid body 52 has a round pot shaped base body 52a, and a reflection body 52b attached to an interior of the base body.

In the state of use shown in FIG. 18, the reflection body 52b is equipped with a concave portion 52b1 that is circularly depressed toward a front at a central portion, and a curved surface portion 52b2 having a ring shaped curved surface which is inclined such that it goes toward a front as it goes to the diameter direction outer side and a rear side becomes convex. The concave portion 52b1 is opposing the front end surface 11a1 of the light guide member 11 in the front and rear direction, and the curved surface portion 52b2 is opposing the ring lens 8 at least in the light emission direction (a direction of the main optical axis LTa shown in FIG. 17).

The reflection body 52b is formed to be solid by a white material. The white material is a fluorocarbon resin, for example. The reflection body 52b functions as a standard replacement for the fruit or vegetable to be measured. Namely, the ring shaped lights LTR (see FIG. 17) emitted from the ring lens 8 are irradiated onto the curved surface portion 52b2 of the reflection body 52b and a part of them enters into the interior. The lights entered into the interior of the reflection body 52b are diffused in the interior and a part of them is emitted from the concave portion 52b1 to the external, and enters into the light guide member 11 as the return lights LTd. The calibration is carried out by the light intensity processing unit CT3 and the central processing unit CT1, such that the measurement result based on these return lights LTd becomes the standard measurement value using the standard lid body 52, that is set in advance and stored in the memory unit CT6.

The measurement with the standard lid body 52 can be carried out by simply putting on the stage portion 2b, as the standard lid body 52 is small and easy to carry around. For this reason, the calibration operation is easy, and a correlation between one individual body and another individual body of the saccharinity meter 51 can be secured easily.

As explained hereinabove, the nondestructive measurement apparatus and nondestructive measurement method according to the present invention make it possible to obtain plural information in the depth direction of the fruit or vegetable and make it possible to obtain internal information even if the skin of the fruit or vegetable is thick. When one wants to know the sugar content of a certain fruit or vegetable, it is sufficient to measure the sugar content of the squeezed juice obtained by squeezing the fruit or vegetable if one only needs to obtain an averaged sugar content value for the entire fruit or vegetable. However, there is a need, among farmers of fruit or vegetable or consumers, to know separately the sugar content of the outer part (skin) of and the sugar content of the inner part (center) of a certain fruit or vegetable, for example. Especially, there is a demand for the farmers of fruits or vegetables for which sweetness has a large impact on the product value, to express the appeal point by numerical value so that one can recognize the sweetness not only at the center portion but also at the outer (skin) portion of the fruit or vegetable. According to the present invention, the above need and demand can be coped with, and there is provided the nondestructive measurement apparatus and method wherein the sugar content distribution inside the fruit or vegetable is obtained in three-dimension by effectively changing the light amount and the measuring points. When obtaining a calibration curve of sugar content for a certain fruit or vegetable, according to the conventional nondestructive measuring apparatus and method, it is necessary to prepare a large amount of fruit or vegetable as samples for measurement. However, some fruits and vegetables, such as watermelons and melons, have a high unit price, and obtaining a calibration curve of sugar content for such high unit price fruits and vegetables requires a large amount of cost to prepare a large number of measurement samples. According to the present invention, when obtaining a calibration curve of sugar content for a certain fruit or vegetable, there is provided a nondestructive measuring apparatus and a non-destructive measuring method which do not require a large number of measurement samples and in which a calibration curve of sugar content based on a small number of measurement samples is obtained.

The embodiments of the present invention are not limited to the configuration and the procedure described above, and may be modified in a range not digressing from an essence of the present invention.

An embodiment of the saccharinity meter 51 may modify the configuration such that the return lights are positively diffused by providing a diffusion portion WB for diffusing the passing lights, on a path of the return lights in which the return lights passed through the fruit or vegetable AS and emitted are passed through the light guide member 11 and emitted from its rear end surface 11fb. Namely, the diffusion portion WB may be provided between a portion enclosed by the ring shaped inner abutting portion 9 on a surface of the fruit or vegetable AS abutted to the inner abutting portion 9 and the rear end surface 11fb of the light guide member 11. Regarding this point, the modified examples 1-3 will be described next.

MODIFIED EXAMPLE 1

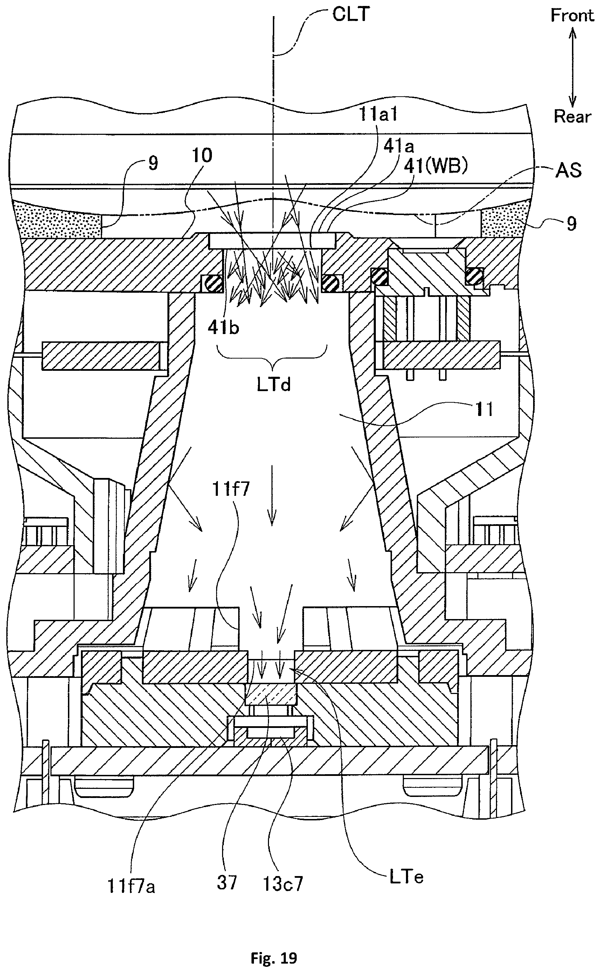

FIG. 19 is a partial cross sectional view for explaining a modified example 1, which is a diagram corresponding to the light guide member 11 and its vicinity in FIG. 17. The modified example 1 is one in which a diffusion plate 41 is arranged in front of the front end surface 11a1 of the light guide member 11, in the saccharinity meter 51 of the embodiment. More specifically, the diffusion plate 41 is attached to the stage base 10 by adhesive or double sided tape or the like, so as to cover an entire surface of the front end surface 11a1 in the front view. The diffusion plate 41 diffuses the lights injected into a front surface 41a and emits them from a rear surface 41b. Any kind of the diffusion plate 41 can be used. For example, it is possible to adopt the known diffusion plate, such as a diffusion plate formed in a plate shape by dispersing and compounding a diffusing agent in a transparent resin, or a diffusion plate in which a microlens is formed on at least one surface of a transparent resin plate, and the like.

In the modified example 1, as shown in FIG. 19, the return lights LTd are diffused and injected into the light guide member 11 from the front end surface 11a1, by the diffusion plate 41 arranged in front of the front end surface 11a1 of the light guide member 11. By means of this, the return lights LTd that have passed through the fruit or vegetable AS and injected into the light guide member 11 are positively diffused to be more highly uniformized by the diffusion plate 41, and injected into the light guide member 11. Then, the uniformized return lights LTd are passing through the light guide member 11 and injected into the photo sensors 13c1-13c7 through the band-pass filters 31-37. For this reason, the correlation coefficient between the absorbance and the Brix value Y is increased, and the measurement precision is improved such that the variations in the absorbance and the Brix value Y at a time of repeatedly measuring the identical fruit or vegetable AS are reduced, for example.

MODIFIED EXAMPLE 2

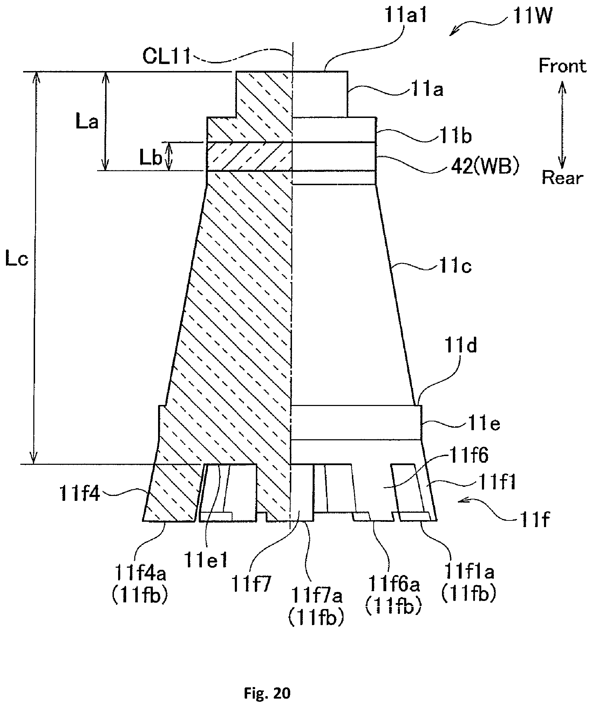

The modified example 2 is one in which the light guide member 11W is applied, instead of the light guide member 11 used in the saccharinity meter 51. FIG. 20 is a half cross sectional view of the light guide member 11W. In detail, the light guide member 11W is one in which a part in a direction of the axial line CL11 in the light guide member 11 is made to be a diffusion member 42. Here, an example in which the light guide member 11W has a diffusion portion WB between the front end surface 11a1 and the rear surface 11e1 of the rear cylinder portion 11e (see FIG. 12) will be described.

In detail, the light guide member 11W has a diffusion member 42 of a thickness Lb, as the diffusion portion WB, with a position separated by a distance La from the front end surface 11a1 to a rear side as the rear end. The diffusion member 42 is formed by dispersing and compounding a diffusing agent in a transparent resin, for example.

In the modified example 2, the return lights LTd (not shown in FIG. 20) injected from the front end surface 11a1 with respect to the light guide member 11W are injected from a front end of the diffusion member 42 and diffused and emitted to a rear side. The maximum value of the distance La can take a distance Lc from the front end surface 11a1 to the rear surface 11e1 of the rear cylinder portion 11e. Namely, the distance La can be set in a range from the thickness Lb to the distance Lc. Also, the thickness of the diffusion member 42 can be set up to the distance La at the maximum.

By means of this, the return lights LTd that passed through the fruit or vegetable AS and injected into the light guide member 11W are positively diffused to be more highly uniformized at a time of passing through the diffusion member 42 provided within the light guide member 11W. After that, the uniformized return lights LTd are emitted from the light guide member 11W, and injected into the photo sensors 13c1-13c7 through the band-pass filters 31-37. For this reason, the correlation coefficient between the absorbance and the Brix value Y is increased, and the measurement precision is improved such that the variations in the absorbance and the Brix value Y at a time of repeatedly measuring the identical fruit or vegetable AS, for example, are reduced.

Moreover, as the modified example 3, the light guide member 11WA formed by resin in which a diffusing agent is dispersed in the same shape may be applied, instead of the light guide member 11 used in the saccharinity meter 51 of the embodiment (see FIG. 12 for reference sign). In this case, the return lights LTd injected into the light guide member 11WA are positively diffused to be more highly uniformized as they propagate within the light guide member 11WA. Then, the uniformized return lights LTd are emitted from the light guide member 11WA, and injected into the photo sensors 13c1-13c7 through the band-pass filters 31-37. For this reason, the correlation coefficient between the absorbance and the Brix value Y is increased, and the measurement precision is improved such that the variations in the absorbance and the Brix value Y at a time of repeatedly measuring the identical fruit or vegetable AS, for example, are reduced.

The modified examples 1-3 can be combined freely within a range in which combinations are possible.

In the embodiment and the modified examples 1-3, the relay lens 18 and the ring lens 8 may be integrated as single optical member. The relay lens 18 and the ring lens 8 may be a plurality of optical members. Namely, they may be an optical system configured by a single optical member or a plurality of optical members, for emitting the lights from the LEDs 14d, in a ring shape from a tip end of the measurement portion K1, and deflecting the emission direction, in a direction of approaching to the light receiving axial line CLT by an angle $\theta$b.

In the embodiment, one in which six types of LEDs 14d with six types among seven types of central wavelengths $\lambda1$-$\lambda7$ set to be their emission central wavelengths are used as the light source group 14dG has been described. Of course, without being limited to this, the LED 14d may be used commonly with respect to the plurality of central wavelengths, in the case where it is judged that lights of the plurality of central wavelengths selected and set for the band-pass filters can be obtained at the optical intensities necessary for the measurement, based on the emission spectrum of the LED 14d that has one emission central wavelength or a broad emission spectrum. Namely, with respect to the m (an integer greater than or equal to 2) types of wavelengths ($\lambda1$-$\lambda m$) selected and set as the central wavelengths of the band-pass filters, q types of LEDs having q ($1 \leq q \leq m$) types of emission central wavelengths respectively may be used. In this case, it is equipped with at least m sets of the photo sensor 13c.

The light source is not limited to the LEDs, and may be other light emission elements. At the time when the amount of light emitted from the light source is changed, an instantaneous change of the light amount is not necessarily needed, and the change of the light amount may be done gradually over time (sweep fashion).

## Claims

1. A nondestructive measurement apparatus for fruit or vegetable, comprising:

   a casing including a grip portion which is held by a hand of an operator, and a measurement portion having a ring shaped abutting portion which is configured to be abutted to a fruit or vegetable;
   a light source group including a plurality of light sources which are arranged separately in a circumferential direction in an interior of said casing;
   a light amount control portion for controlling the light amount emitted from said light source group;
   a ring lens arranged in a ring shape smaller than an inner portion surrounded by said abutting portion, for emitting light coming from said light source group toward an outside of said casing as ring shape light as a ring shaped beam;
   a light guide member having one end surface exposed to an inner side of said ring lens and the other end surface positioned in the interior of said casing, for emitting the light entered from said one end surface to the outside from said the other end surface;
   a photo sensor arranged inside said casing, for detecting light emitted from said the other end surface of said light guide member; and
   a light intensity processor for obtaining absorbance according to light intensity detected by said photo sensor, wherein said light source group is controlled by said light amount control portion to emit light of at least two different light amount, and
   wherein said light intensity processor obtain a calibration curve of sugar content of said fruit or vegetable based on the absorbance of said at least two different light amount and Brix value of said fruit or vegetable in a depth direction corresponding to said light amount.

2. A nondestructive measurement apparatus for fruit or vegetable according to claim 1, wherein said light intensity processor obtains a distribution of Brix value in the depth direction of said fruit or vegetable based on absorbance of said at least two light amount of light.

3.  A nondestructive measurement apparatus for fruit or vegetable according to claim 2, further comprising an electric circuit including an electrolytic capacitor, wherein said electrolytic capacitor is charged by a power source while said light source group does not emit light, and electric current is supplied to said light source group from both said power source and electrolytic capacitor while said light source group emits light.

4.  A nondestructive measurement apparatus for fruit or vegetable according to any one of said claims 1-3, further comprising a ring shaped relay lens between said light source group and said ring lens, for guiding the light from said light source group to said ring lens.

5.  A nondestructive measurement apparatus for fruit or vegetable according to any one of claims 1-3, wherein main optical axis of ring shaped light beam emitted from said ring lens contracts after the light are emitted from said ring lens.

6.  A nondestructive measurement apparatus for fruit or vegetable according to any one of said claims 1-3, wherein said photo sensor is formed by at least m sets of photo sensors (where m is an integer greater than or equal to 2), and said nondestructive measurement apparatus further comprising band-pass filters, each having a respective one of m types of different wavelengths $\lambda1-\lambda m$ as a central wavelength, between the respective one of the m sets of said photo sensors and said the other end surface of said light guide member.

7.  A nondestructive measurement apparatus for fruit or vegetable according to claim 6, wherein said light intensity processor obtains absorbance according to the detected light intensities corresponding to said wavelengths $\lambda1-\lambda m$ obtained by said m sets of photo sensors, and calculates a Brix value from the obtained absorbance.

8.  A nondestructive measurement apparatus for fruit or vegetable according to any one of said claims 1-6, wherein said grip portion has a longitudinal handle which is holdable by a hand of an operator, and said measurement portion is formed such that an extending direction of said outer abutting portion is a direction along said handle, at said one end portion of the handle in said grip portion, and that a tip end portion of said outer abutting portion is positioned to protrude from a surface of said grip portion.

9.  A nondestructive measurement apparatus for fruit or vegetable according to any one of said claims 1-3, further comprising a light diffusion member between said the other end surface of said light guide member and a surface of the fruit or vegetable surrounded by said abutting portion while said abutting portion and said fruit or vegetable are kept in contact under measurement, for diffusing the light passing therethrough

10. A nondestructive measurement method for fruit or vegetable, comprising the steps of:

    a step of contacting fruit or vegetable to be measured to a measurement portion;
    a step of irradiating light onto said fruit or vegetable so that light emitted from a light source group formed by a plurality of light sources enters inside said fruit or vegetable; a step of receiving, by a photo sensor, light outputted from said fruit or vegetable after subjected to diffused reflection and partial absorption in the fruit or vegetable, due to the internal condition of the fruit or vegetable; and
    a step of processing light intensity to obtain absorbance based on light intensity detected by said photo sensor, wherein said step of irradiating light onto the said fruit or vegetable is performed in a multiple mode such that at least two different light amounts are emitted, and
    wherein said step of processing light intensity includes a step of obtaining a calibration curve of sugar content of said fruit or vegetable, based on absorbance of at least two different light amounts and the Brix value in depth of said fruit or vegetable corresponding to said light amount.

11. A nondestructive measurement method for fruit or vegetable according to claim 10, wherein said step of processing light intensity comprises a step of obtaining a distribution of Brix value in the depth direction of the fruit or vegetable, based on absorbance of said at least two different light amounts.

12. A nondestructive measurement method for fruit or vegetable according to claim 11, wherein said light irradiating step in multiple mode includes a step of charging an electrolytic capacitor from a power source during non-irradiation state and providing currents to said light source group from both said power source and electrolytic capacitor during irradiation state.

**Fig. 1**

**Fig. 2**

Fig. 3

**Fig. 4**

Fig. 5

**Fig. 6**

Fig. 7

Fig. 8

**Fig. 9**

**Fig. 10**

CL11

11a1

11a

11

11b

11c

11d

11e

11f2

11f

11f2a

11f6a    11f6    11f1

11f1a

Front

Rear

Fig. 11

**Fig. 12**

Fig. 13

**Fig. 14**

Up
↕
Down

AS

K1

51

91

Fig. 15

**Fig. 16**

**Fig. 17**

Fig. 18

Fig. 19

Fig. 20

**Fig. 21**

**Fig. 22**

**Fig. 23**

Fig. 24

**Pre-charge**

DC-DC Converter

Current Restriction Resistor

Reverse Current Prevention Diode

Switch Off

Electrolytic Capacitor

LED

**Light Emitting**

DC-DC Converter

Current Restriction Resistor

Reverse Current Prevention Diode

Switch On

Electrolytic Capacitor

LED

EP 4 560 295 A1

44

## INTERNATIONAL SEARCH REPORT

| International application No. |
|---|
| **PCT/JP2024/019174** |

**A. CLASSIFICATION OF SUBJECT MATTER**

*G01N 21/3563*(2014.01)i; *G01N 21/27*(2006.01)i; *G01N 21/359*(2014.01)i
FI: G01N21/3563; G01N21/27 F; G01N21/359

According to International Patent Classification (IPC) or to both national classification and IPC

**B. FIELDS SEARCHED**

Minimum documentation searched (classification system followed by classification symbols)

G01N21/00-G01N21/61; G01N21/84-G01N21/958

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 2006-226775 A (UNIV TOYOHASHI TECHNOLOGY) 31 August 2006 (2006-08-31) entire text, all drawings | 1-12 |
| A | WO 2018/047366 A1 (ATAGO CO., LTD.) 15 March 2018 (2018-03-15) entire text, all drawings | 1-12 |
| A | JP 8-050093 A (KURASHIKI BOSEKI K.K.) 20 February 1996 (1996-02-20) entire text, all drawings | 1-12 |
| A | JP 2007-523731 A (NELLCOR PURITAN BENNETT INCORPORATED) 23 August 2007 (2007-08-23) entire text, all drawings | 1-12 |
| A | WO 2013/137145 A1 (CHIYODA ELECTRONICS CO., LTD.) 19 September 2013 (2013-09-19) entire text, all drawings | 1-12 |

☐ Further documents are listed in the continuation of Box C.　　☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **10 June 2024** | **18 June 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

## INTERNATIONAL SEARCH REPORT
### Information on patent family members

International application No.

**PCT/JP2024/019174**

| Patent document cited in search report | | | Publication date (day/month/year) | Patent family member(s) | | | Publication date (day/month/year) |
|---|---|---|---|---|---|---|---|
| JP | 2006-226775 | A | 31 August 2006 | (Family: none) | | | |
| WO | 2018/047366 | A1 | 15 March 2018 | US | 2019/0003963 | A1 | |
| | | | | entire text, all drawings | | | |
| | | | | EP | 3511698 | A1 | |
| | | | | CN | 108291869 | A | |
| | | | | IL | 259443 | A | |
| | | | | KR | 10-2019-0047654 | A | |
| | | | | TW | 201812276 | A | |
| JP | 8-050093 | A | 20 February 1996 | (Family: none) | | | |
| JP | 2007-523731 | A | 23 August 2007 | US | 2005/0187449 | A1 | |
| | | | | entire text, all drawings | | | |
| | | | | WO | 2005/082238 | A1 | |
| | | | | EP | 1722672 | A1 | |
| | | | | CA | 2556719 | A1 | |
| | | | | KR | 10-2006-0134091 | A | |
| | | | | CN | 1953699 | A | |
| | | | | AU | 2005216974 | A1 | |
| WO | 2013/137145 | A1 | 19 September 2013 | JP | 2015-108508 | A | |
| | | | | entire text, all drawings | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002014042 A

- WO 2018047366 A